# FASCICULE DE BREVET EUROPEEN

(11) **EP 2 932 431 B1**
(45) Date de publication et mention de la délivrance du brevet: **27.11.2019**
(21) Numéro de dépôt: 13821064.6
(22) Date de dépôt: 13.12.2013
(51) Int. Cl.: G06F 21/62, G16H 10/60

(54) **PROCEDE D'ACCES SECURISE A DES DONNEES MEDICALES CONFIDENTIELLES, ET SUPPORT DE STOCKAGE POUR LEDIT PROCEDE**
VERFAHREN FÜR SICHEREN ZUGANG ZU VERTRAULICHEN MEDIZINISCHEN DATEN UND SPEICHERMEDIUM FÜR DIESES VERFAHREN
METHOD OF SECURE ACCESS TO CONFIDENTIAL MEDICAL DATA, AND STORAGE MEDIUM FOR SAID METHOD

(30) Priorité: 13.12.2012 FR 1203396; 31.01.2013 FR 1300205; 21.06.2013 FR 1301457
(43) Date de publication de la demande: 21.10.2015
(73) Titulaire: SCP MRC 2019, 98000 Les Bougainviliers (MC)
(72) Inventeur: COUDERT, Patrick, 06190 Roquebrune Cap Martin (FR)
(74) Mandataire: Schuffenecker, Thierry
(86) Numéro de dépôt international: PCT/EP2013/003772
(87) Numéro de publication internationale: WO 2014/090411

(56) Documents cités:
- WO-A1-01/06468
- US-A1- 2001 041 991
- US-A1- 2004 186 746
- US-A1- 2005 197 859

## Description

### Domaine technique de l'invention

La présente invention concerne les systèmes d'information, et notamment un procédé d'accès sécurisé à des données médicales confidentielles, notamment pour la consultation d'un dossier ou profil médical d'urgence.

### Etat de la technique

Avec l'essor des moyens de déplacement modernes, la mobilité est à présent au cœur des préoccupations de nos contemporains du 21^{ème} siècle. Avec le besoin qui en résulte de dématérialiser significativement des informations importantes qui le concernent, besoin d'autant plus critique que leur détenteur peut se trouver fort loin de son domicile.

Cette problématique se pose bien évidemment pour les informations médicales concernant tout un chacun, et susceptible de venir constituer un dossier médical propre à son titulaire, auquel ce dernier doit pouvoir avoir accès quel que soit le lieu géographique où il se situe mais également quelles que soient les conditions dans lesquelles cet accès est sollicité.

Les déposants de la présente demande de brevet ont développé différentes techniques visant à développer le concept de Dossier Médical numérique - qu'il soit anonyme ou non - permettant à un citoyen/patient potentiel d'un service médical public ou privé d'utiliser pleinement un dossier médical numérique enrichi de données médicales le concernant.

La demande de brevet n° 08368018.1 du 19 Septembre 2008 (Publication EP2166484), intitulée « *Procédé d'accès à des données nominatives, tel qu'un dossier médical personnalisé, à partir d'un agent local de génération* » et dont les demandeurs de la présente demande sont à l'origine, décrit une première technique visant à dématérialiser le dossier médical d'un patient venant consulter un groupe de thérapeutes. A cet effet des procédures spécifiques sont mises en oeuvre pour assurer un stockage anonyme sur un serveur dit DMA (Dossier Médical Anonyme), lequel stockage sert, lors d'une consultation du patient chez un praticien, à construire, au sein même du cabinet du praticien, une instance du Dossier Médical Nominatif - ou personnalisé - du patient et ce en préservant le caractère confidentiel des informations hautement sensibles que comporte ce dossier médical. De cette manière, il est permis au patient d'aller consulter divers praticiens, notamment au sein d'un même groupement professionnel mais situés en des lieux physiquement éloignés et qui pourront accéder partout au dossier médical personnalisé sans risque que les prestataires informatiques intervenant dans la transmission de l'information puissent briser la chaîne du secret.

On parvient ainsi à préserver la confidentialité des informations stockées sur le réseau Internet. La solution qui se trouve décrite dans cette demande précitée apporte ainsi une amélioration significative au service rendu au patient qui, toutefois, reste confiné dans le sein du cabinet du praticien et prend fin dès lors que le patient prend congé de son praticien.

La demande de brevet français 11/02726 en date du 8 Septembre 2011, intitulée *"Procédé d'accès et de partage d'un dossier informatique enrichi par des ressources multimédias personnalisées"*, non publiée à la date de dépôt de la présente et déposée par les inventeurs de la présente demande de brevet décrit un perfectionnement permettant au patient d'accéder très librement à son dossier médical personnalisé, enrichi par de nombreuses références à des ressources judicieusement choisies par le praticien.

La demande de brevet français 12/00907 en date du 27 Mars 2012, intitulée *"Procédé d'accès et de partage d'un dossier médical"* non publiée à la date de dépôt de la présente et déposée par les inventeurs de la présente demande de brevet décrit un perfectionnement permettant de faciliter le portage du dossier médical entre plusieurs praticiens appartenant ou pas à un même groupement professionnels, voire à un même pays.

La demande de brevet français 12/02401 en date du 10 Septembre 2012, intitulée « *Procédé d'accès et de partage d'un dossier médical* » décrit un procédé permettant une consultation à distance entre un patient et son praticien, tout en autorisant un accès sécurisé à un dossier médical partagé, nominatif ou non, hébergé sur un serveur tiers. La consultation menée à distance entre le patient et son praticien permet la validation par le médecin de nouvelles données médicales en vue de leur stockage sur le dossier médical partagé et, par suite, la mise à jour de ce dernier.

Ces demandes de brevets apportent déjà des améliorations significatives au problème du développement d'un véritable dossier médical numérique et susceptible de servir d'assise à une politique moderne de Santé Publique.

Pour autant, si les techniques évoquées précédent permettent aux patients et à leurs médecins la constitution, la mise à jour et l'accès à un dossier médical numérique dématérialisé, un problème crucial demeure qui est celui de gérer la situation d'urgence dans laquelle tout un chacun peut se trouver, lorsque notamment l'on n'est pas en capacité d'accéder à son dossier médical numérique. En effet, dans une situation d'état de choc ou d'inconscience, le titulaire n'est plus en mesure d'accéder à son dossier médical dématérialisé et ne pourra pas en tirer avantage, alors même qu'il se sera astreint à le constituer, patiemment au fil des années, dans l'espoir qu'il pourra lui servir le jour où son pronostic vital sera engagé.

Comme on le voit, il est critique qu'un dossier médical dématérialisé puisse servir dans toutes les circonstances envisageables, et notamment celle d'une situation d'urgence dans laquelle le patient est en état de choc voire même inconscient.

Bien évidemment, l'on pourrait imaginer que le patient conserve dans son portefeuille les codes et clés d'accès à son dossier médical numérique dans l'espoir que ces clés puissent servir dans une situation d'urgence.

Mais cette solution - simple dans son application - pose clairement un problème de sécurité car le vol du portefeuille expose son propriétaire à la divulgation sans limite des données sensibles que comporte son dossier médical.

C'est là un des problèmes auxquels la présente invention apporte une solution.

Un autre problème consiste également à pouvoir répondre parfaitement aux exigences réglementaires des différentes lois informatiques et liberté de chaque pays et notamment des pays les plus élaborés en matière de législation comme ceux appartenant à la convention 108 dont la France fait partie avec son expression juridique ; la CNIL

Il est donc hautement souhaitable de pouvoir sécuriser les informations échangées entre un serveur centralisé et sécurisé (par un hébergement répondant à un niveau de protection adéquat de chaque pays), et un support externe de type carte ou/et clé USB, Il est particulièrement important que cet échange soit conforme aux exigences réglementaires nationales, dans la plupart des pays, et en particulier dans les pays dont la législation délivre un très haut niveau de protection juridique des données personnelles et/ou de santé.

L'état de la technique comporte les antériorités suivantes :
US2001/041991 décrit un procédé et un système pour fournir un service de gestion de dossier médical qui prend en charge la création, le stockage, l'accès, la mise à jour, et la distribution des dossiers médicaux des patients, en particulier l'imagerie médicale de diagnostic de qualité, sous le contrôle d'un patient et la prise en charge coordonnée du patient et son médecin.

WO 01/06468 décrit un système pour l'enregistrement du suivi des soins d'un patient, pour le stockage, la gestion et la récupération des informations de soins de santé par l'intermédiaire d'une carte à puce, ainsi qu'une application de carte à puce ayant une interface pouvant fonctionner pour convertir au moins un fichier stocké sur la carte à puce dans un fichier de base de données de carte à puce de base de données. En outre, le système comprend un fichier de base de données de système qui comprend des informations de santé des patients. Le fichier de base de données de carte à puce est un sous-ensemble du fichier de base de données du système. Le fichier de base de données de carte à puce est capable d'être lu / écrit, et peut contenir un code de sécurité pour accéder au fichier de base de données du système.

US 2005/197859 décrit un procédé et dispositif pour la fourniture et l'utilisation d'un système de recherche électronique portable de stockage de données et d'information pour la santé. Chaque périphérique de stockage portable (PSD) (par exemple, carte à puce) pour un groupe, par exemple, des ménages, permet le stockage et la récupération de l'information au niveau des ménages en plus des données de membres de chaque ménage / information. Les données / informations sont stockées correspondant à différentes applications. Le PSD est accessible par le système informatique d'un fournisseur de services.

US2004/186746 décrit un système et un procédé pour le stockage, la gestion de transport et de communication santé et l'information médicale

### Exposé de l'invention

La présente invention a pour objet de réaliser un procédé d'accès à un dossier médical nominatif et confidentiel qui puisse être utilisé dans une grande variété de situations, et tout spécifiquement dans les situations d'urgence dans lesquelles un patient se trouve dans l'incapacité d'accéder lui-même à son dossier.

Elle a aussi pour objet de permettre à tout un chacun de constituer un Profil Médical destiné à une situation d'urgence, (ou à mettre en avant des problèmes de santé particulier non spécifiquement liés à l'urgence), grâce à une validation médicale de plusieurs niveaux (questionnaire médicalement pertinent, pièces jointes médicales, validation secondaire du praticien...).

La présente invention a également pour objet d'offrir à tout un chacun la possibilité de publier ou de rendre confidentielle chacune des informations médicales auto-renseignées.

La présente invention a pour autre objet de réaliser un procédé d'accès à un dossier médical nominatif permettant de renforcer l'efficacité des services d'urgences appelés à prendre en charge les patients.

Par ailleurs, la présente invention a pour objet de réaliser une nouvelle carte médicale susceptible de stocker un dossier médical d'urgence non nominatif et non confidentiel, et permettant un accès ultérieur à des données plus sensibles au moyen d'un procédé sécurisé.

Plus largement, ce procédé peut-être étendu à des situations autres que celles de l'urgence afin de véhiculer à travers le patient un profil médical pertinent pour tout praticien, qu'il soit urgentiste ou non.

L'invention réalise ces buts au moyen d'un procédé de génération d'un dossier médical numérique stocké sur un serveur sécurisé et accessible depuis un premier système via un réseau de communication, le dossier médical numérique comportant des données nominatives et confidentielles.

Le procédé comporte la possibilité de générer automatiquement un profil médical d'urgence, PMU, dénué de toute information nominatives et confidentielles, publiable directement sans mot de passe et/ou permettant un accès indirect au dossier médical numérique confidentiel ou nominatif via un login/mot de passe ou un tiers de confiance.

Dans un mode de réalisation particulier, le dossier médical d'urgence, PMI, est généré lors de l'ouverture du compte du titulaire, à la suite de la saisie d'un questionnaire médical prédéterminé. De préférence, la saisie est associée à l'attribution d'un caractère confidentiel ou non à chacune des informations saisies par le titulaire du dossier médical.

Dans un mode de réalisation particulier, le dossier médical d'urgence, PMI, est stocké sur un support physique détenu par le titulaire, telle une carte mémoire ou une clé USB, permettant un accès sans restriction auxdites informations non confidentielles saisies par le titulaire. De préférence, le contenu du support physique est protégé par signature électronique de manière à garantir l'intégrité des données y stockées. En particulier, le support comporte un lien vers ledit serveur sécurisé permettant un accès aux données nominatives et confidentielles stockées sur ledit serveur via une procédure de vérification de mot de passe.

Plus spécifiquement, le procédé comporte les étapes :
- création d'un compte et génération d'un identifiant et d'un mot de passe ;
- création d'un questionnaire administratif comportant des informations administrative nominatives ;
- création d'un questionnaire médical destiné à collecter des données d'ordre médical, éventuellement accompagné d'annexes ou de pièces jointes validant ces données ;
- génération d'une synthèse des données saisies via le questionnaire médical, en relation avec un outil de classement destiné à assigner à chacune des données un caractère confidentiel ou non ;
- désignation d'un ou plusieurs tiers de confiance ;
- génération automatique d'un Profil Médical d'Urgence, PMU, ne comportant que des données non nominatives et classifiées non confidentielles, ledit Profil Médical d'Urgence étant stocké sur une zone non protégée et accessible depuis un support externe comportant ledit identifiant, les autres données nominatives et/ou confidentielles étant stockées dans une zone protégées dudit serveur sécurisé accessible au moyen de l'identifiant et du mot de passe ;
ledit Profil Médical d'Urgence comportant en outre un lien/moyen permettant d'accéder à un tiers de confiance en vue de l'obtention d'une clé de substitution utilisable en l'absence d'un mot de passe.

Dans un mode de réalisation particulier, la création d'un questionnaire administratif comporte en outre l'attribution, pour l'un ou plusieurs des éléments saisies dans ledit questionnaire administratif, d'une fonction d'identification de substitution permettant un accès ultérieur au dossier médical sans la détention de l'identifiant du compte.

Dans un autre mode de réalisation particulier, le Profil Médical d'Urgence, PMU, est stocké sur une carte électronique dotée d'un port USB, et attribuée au titulaire dudit compte.

Dans un mode de réalisation particulier, la carte électronique sert de stockage en outre à des données qui pourront être synchronisées avec ledit serveur sécurisé.

Dans un autre mode de réalisation, les données nominatives et/ou confidentielles sont accessibles à partir de la connaissance de l'identifiant attribué au titulaire et du mot de passe correspondant.

De préférence, les données nominatives et/ou confidentielles sont accessibles à partir de la connaissance de l'identifiant attribué au titulaire et de ladite clé de substitution, et après l'authentification du système requérant l'accès aux données nominatives et/ou médicales, comme appartenant bien à un service répertorié comme étant un service d'urgence (ou à un médecin placé en situation d'urgence). L'invention permet également la réalisation d'un procédé d'accès à un dossier médical numérique stocké sur un serveur sécurisé, légal et accessible depuis un premier système via un réseau de communication, le dossier médical numérique comportant des données nominatives et confidentielles.

Le procédé comporte :
- l'affichage d'un profil médical d'urgence, PMU, dénué de toute information nominatives et confidentielles et permettant un accès indirect au dossier médical numérique via un tiers de confiance ;
- l'accès audit dossier médical numérique via un ou plusieurs tiers de confiances dotés chacun d'une clé de substitution ou de tout autre moyen technique de déverrouillage. Dans un mode de réalisation particulier, l'accès au dossier médical découle de la réception par le serveur sécurisé d'un message électronique transmis depuis le propre système du tiers de confiance, le message électronique constituant la clé de substitution permettant l'accès au serveur sécurisé.

De préférence, le message électronique émis par le tiers de confiance, et jouant le rôle de la clé de substitution , est un SMS ou un courriel ou un message transmis depuis une application installée depuis le propre système du tiers de confiance.

Alternativement, l'ouverture du dossier médical numérique pourra résulter de la réception par ledit serveur sécurisé d'un appel vocal reçu de la part dudit tiers de confiance.

Plus spécifiquement, le procédé comporte en outre les étapes suivantes :
- accès au serveur sécurisé manuellement ou automatiquement, éventuellement au moyen d'un support physique comportant un identifiant,
- saisie de l'identifiant (si celui-ci n'a pas été transféré automatiquement)
- ouverture d'une session avec le serveur sécurisé et affichage du Profil Medical d'Urgence ou PMU
- possibilité d'accéder à des données nominatives et/ou confidentielles en saisissant un mot de passe et, en cas de mot de passe valide, ouverture du dossier médical ;
- en cas de défaut de mot de passe valide, transmission optionnelle d'un courriel au titulaire du compte pour informer le titulaire d'une éventuelle tentative d'accès frauduleux
- sans mot de passe mise en place d'un système de sécurité dit de « tiers de confiance » qui permettra d'autoriser l'accès;
   - identification d'un ou des tiers de confiance;
   - contact d'un ou des tiers de confiance et obtention d'un accord d'accès (clé de substitution;ou tout autre moyen)
   - validation par le serveur sécurisé de cet accord ;
   - l'autorisation est délivrée par le serveur sécurisé au service demandeur soit par une clé de substitution (après vérification de ladite clé de substitution et, en cas de concordance avec les clés inscrites dans le dossier médical, ouverture du dossier médical), soit par tout autre moyen et en particulier par la délivrance d'un mot de passe aléatoire et temporaire automatiquement délivré par le serveur. L'envoi de cette information au service/praticien demandeur se fera par tout moyen technique (email, sms, application smartphone...)- cette autorisation permet ainsi au service/praticien d'accéder aux données privées nominatives et/ou confidentielles

Dans le cas d'une utilisation de la la clé de substitution du tiers de confiance, celle-ci pourra, de préférence, correspondre à l'identifiant de la propre carte du tiers de confiance.

Dans un mode de réalisation particulier, l'accès au service utilisera les étapes :
- insertion et lecture d'une carte non nominative comportant un Profil Médical d'Urgence, PMU, ne comportant aucune information médicales et aucune information confidentielle, ainsi qu'un lien permettant un accès audit serveur ainsi qu'à un ou plusieurs tiers de confiance ;
- Affichage du Profil Médical d'Urgence sur un dispositif de traitement de données dudit dossier médical d'urgence ;

L'invention permet également la réalisation d'un support de stockage, telle qu'une carte médicale, comportant des circuits électroniques et des moyens de stockage pour le Profil Médical d'Urgence.

Il est précisé que pour respecter au maximum le caractère anonyme de cette carte médicale, des critères de sécurité supplémentaires peuvent être rajoutés :
- La carte, elle-même, est destinée à ne contenir aucune information à caractère nominatif afin de ne pouvoir lier (en dehors d'un acte délictueux) les informations médicales et à caractère personnel avec son patient/auteur.
- le contenu de cette carte est destiné à rester anonyme et elles auront été validées par le patient comme « publiables ».
- Concernant d'éventuelles pièces jointes il sera proposé au patient lors de la scanérisation, d'enlever toute information nominative, soit par un système de stick auto-collant, soit par un système de brouillage afin de permettre une publication « anonymisée » sur le document de synthèse.
- Le système permettra d'identifier ce qui est nominatif de ce qui ne l'est pas et donc d'inclure ou non dans ce document de synthèse la pièce jointe. Celle-ci sera de toute manière accessible lors de l'ouverture du dossier confidentiel sur le serveur.
- Des signes distinctifs permettront à un urgentiste de créer un lien entre patient et la carte (taille, poids, âge, couleur des yeux, cicatrices tatouages...)
- Cette carte sera délivrée avec numéro réglementaire déterminé selon un algorithme (dont une copie en annexe est mise à titre d'exemple), ce numéro devant être protégé visuellement pour éviter tout risque de visualisation intempestive (pochette + emballage opaque...).

Si le système de stockage se fait par l'intermédiaire d'une clé USB (mais d'une manière générale pour tout support externe numérique) il conviendra de s'assurer de la bonne intégrité des données déposées sur la clé
- Ainsi la sécurisation du système devra empêcher tout enregistrement de document autre que celui en provenance du serveur sécurisé (lien IP avec le serveur agréé, cryptage de zone ou de fichier)
- Un protocole de sécurité de maintien de l'intégrité des données sera mis en place lors de la génération du PDF et de son transfert sur la clé. Un blocage/cryptage de zone ou de fichier permettra d'interdire l'enregistrement de tout document ne provenant pas du serveur sécurisé (impossibilité par le patient de déposer tout fichier ou document issu de son ordinateur sans que celui-ci n'ait transité par le serveur sécurisé. Un système de protection de virus permettra d'éviter de porter atteinte à la sécurité des données sur le support externe / USB ou autre.

Par ailleurs, l'invention permet également de gérer l'importante question des échanges transfrontaliers entre pays de protection juridique non équivalente :
- Seules les données contenues sur cette carte (puisque non nominatives et/ou décrétées publiables par le patient) seront accessibles depuis un pays dont la législation n'offre pas un niveau de sécurité adéquat.
- Concernant les données nominatives ou confidentielles en ligne, seules la visualisation sera possible. Le système interdira tout transfert de données entre pays dont la législation n'offre pas un système de protection équivalent.
- Un système de contrôle automatique à travers le serveur sécurisé permettra d'identifier par une reconnaissance d'adresse IP (ou tout autre moyen comme la reconnaissance de la langue du navigateur internet...), le pays qui demande le transfert de données et sera en capacité automatiquement à bloquer ou autoriser le transfert dans un sens ou dans l'autre, suivant le niveau de législation légalement autorisé.

L'invention réalise ces buts au moyen d'un procédé de génération d'un dossier médical numérique destiné à être stocké sur un support amovible, tel qu'une carte à mémoire, comportant un espace de stockage ainsi qu'un programme informatique résidant sur ledit support, ledit procédé comportant les étapes:
- transmission d'une requête destinée à un serveur externe dans le but d'obtenir une clé ou un code d'activation dudit programme informatique, ladite requête permettant un enregistrement administratif auprès dudit serveur;
- réception de la clé ou dudit code d'activation généré par ledit serveur ;
- démarrage dudit programme informatique permettant la saisie par le patient d'un dossier Questionnaire médical conçu de manière à permettre la collecte de ses données médicales personnelles, et le stockage de celles-ci dans une base de données disposée au sein dudit support de stockage, la saisie étant associée à l'attribution d'un caractère confidentiel ou non à chacune des informations saisies par le titulaire du dossier médical;
- génération automatique d'un profil médical personnel d'urgence correspondant au patient, par exemple dans un format électronique pouvant être disponible en plusieurs langues, ne comportant que les seules données auxquelles ont été attribuées un caractère non confidentiel.

Dans un mode de réalisation particulier, le support comporte un lien vers ledit serveur sécurisé permettant un accès aux données nominatives et confidentielles stockées sur ledit serveur via une procédure de vérification de mot de passe.

De préférence , le procédé comporte les étapes :
- création d'un compte et génération d'un identifiant et d'un mot de passe ;
- création d'un questionnaire administratif comportant des informations administrative nominatives ;
- création d'un questionnaire médical destiné à collecter des données d'ordre médical, éventuellement accompagné d'annexes ou de pièces jointes validant ces données ;
- génération d'une synthèse des données saisies via le questionnaire médical, en relation avec un outil de classement destiné à assigner à chacune des données un caractère confidentiel ou non ;
- désignation d'un ou plusieurs tiers de confiance ;
- génération automatique d'un Profil Médical d'Urgence, PMU, ne comportant que des données non nominatives et classifiées non confidentielles, ledit Profil Médical d'Urgence étant stocké sur une zone non protégée et accessible depuis un support externe comportant ledit identifiant, les autres données nominatives et/ou confidentielles étant stockées dans une zone protégées dudit serveur sécurisé accessible au moyen de l'identifiant et du mot de passe ;
ledit Profil Médical d'Urgence comportant en outre un lien permettant d'accéder à un tiers de confiance en vue de l'obtention d'une clé de substitution utilisable en l'absence d'un mot de passe ou de tout autre moyen technique permettant la lecture du dossier médical confidentiel et/ou nominatif

Dans un mode de réalisation particulier, le procédé est caractérisé en ce que la clé de substitution résulte d'une expression de volonté dudit tiers de confiance de permettre l'accès au dossier médical, prenant la forme d'un message électronique, tel que SMS ou courriel, ou d'un appel vocal, ou d'un message électronique transmis par une application depuis le système dudit tiers vocal.

L'invention permet également la réalisation d'un support de stockage électronique pour un dossier médical numérique comportant un espace de stockage pour ledit dossier médical numérique, ainsi qu'un programme informatique résidant sur ledit support. Le support comporte en outre :
- des moyens de transmission d'une requête destinée à un serveur externe dans le but d'obtenir une clé ou un code d'activation dudit programme informatique, ladite requête permettant un enregistrement administratif auprès dudit serveur;
- des moyens de réception de la clé ou dudit code d'activation généré par ledit serveur ;
- des moyens pour démarrer ledit programme informatique permettant la saisie par le patient d'un dossier Questionnaire médical conçu de manière à permettre la collecte de ses données médicales personnelles, et le stockage de celles-ci dans une base de données disposée au sein dudit support de stockage, la saisie étant associée à l'attribution d'un caractère confidentiel ou non à chacune des informations saisies par le titulaire du dossier médical;
- des moyens pour générer automatiquement un profil médical personnel d'urgence correspondant au patient, par exemple dans un format électronique pouvant être disponible en plusieurs langues, ne comportant que les seules données auxquelles ont été attribuées un caractère non confidentiel.

Dans un mode de réalisation particulier, le support comporte des moyens permettant un accès indirect au dossier médical numérique confidentiel et/ou nominatif via un tiers de confiance.

De préférence, le support comporte un lien vers ledit serveur sécurisé permettant un accès aux données nominatives.

De préférence, le support est une carte à mémoire ou une clé de type USB.

Dans un mode de réalisation particulier, le programme informatique présent sur le support est un applicatif de gestion du questionnaire puisse être intégré au support externe (USB ou autre type). Il s'agit alors d'un « applicatif embarqué » sur support externe local. Celui-ci répondrait toutefois à la même logique de séparation des données patients en deux catégories : les données déclarées comme publiables, et celles devant être enregistrées comme confidentielles et accessibles par un login/passeword (« coffre fort sécurisé »).
Ce logiciel programmé de manière spécifique permettrait ainsi, à tout un chacun, de déterminer lui-même ses informations publiables ou confidentielles, voire nominatives ou non. (photo, identité...)
Dans ce cas particulier, la validation médicale peut se faire également avec l'adjonction de pièces jointes scannées qui authentifient les informations saisies (dans un mode particulier, une validation médicale secondaire avec un système d'authentification peut être rajouté dans le cas d'une assistance médicale lors du remplissage des informations).
Dans ce mode de réalisation, le lien avec le serveur centralisé permet d'initialiser la carte, de délivrer les mots de passe qui protègent les données confidentielles, de délivrer un nouveau mot de passe en cas d'oubli, et surtout offre une sécurité essentielle qui permet le blocage de l'ouverture confidentielle en cas de perte ou de vol.

Un système de sécurisation et de protection du support externe doit permettre entre autres, de crypter le logiciel embarqué et ses données, de protéger l'accès à des virus, ...)
Ce mode de réalisation qui utilise un logiciel local embarqué sur le support externe, en lien avec un serveur de sécurisation, conserve pour avantage de donner totalement le libre arbitre au patient en respectant l'esprit du législateur sur la protection des données médicales. Chacun peut ainsi juger quelles données médicales lui paraissent essentielles et à faible degré de confidentialité pour être publiables en situation d'urgence, de celles qui ont une priorité de confidentialité.

La notion de tiers de confiance serait toujours présente et utile pour l'accès aux données médicales confidentielles en cas d'incapacité.

### Description des dessins

D'autres caractéristiques, but et avantages de l'invention apparaîtront à la lecture de la description et des dessins ci-après, donnés uniquement à titre d'exemples non limitatifs. Sur les dessins annexés :
La figure 1 illustre la procédé de création de compte et de constitution du dossier médical stocké sur un serveur distant sécurisé dans un premier mode de réalisation de l'invention.
La figure 2 représente un premier mode de réalisation d'un procédé d'accès à un dossier médical.
La figure 3 illustre un second mode de réalisation d'un procédé d'accès à un dossier médical, plus sécurisé que le premier mode.
La figure 4 illustre une architecture générale représentant le système d'un médecin urgentiste et celui d'un tiers de confiance permettant une procédure automatisée pour l'accès au dossier médical.
La figure 5 illustre un procédé suivant un troisième mode de réalisation, permettant une obtention automatique d'une clé de substitution gérée par le serveur sécurisé distant.
La figure 6 illustre un mode de réalisation d'une carte ayant vocation au stockage d'un dossier médical d'urgence, suivant un quatrième mode de réalisation.
La figure 7 illustre un procédé d'accès au DM suivant un quatrième mode de réalisation, dans lequel l'obtention de la clé de substitution est directement géré depuis le serveur sécurisé distant.
La figure 8 illustre un procédé d'accès au DM suivant un cinquième mode de réalisation dans lequel le système requérant du médecin urgentiste gère directement la procédure d'obtention de la clé de substitution.
La figure 9 illustre un sixième mode de réalisation montrant la gestion locale du dossier médical personnalisé sur un support externe.
La figure 10 est un diagramme illustratif du procédé de la figure 9.

### Description des modes de réalisation préférés

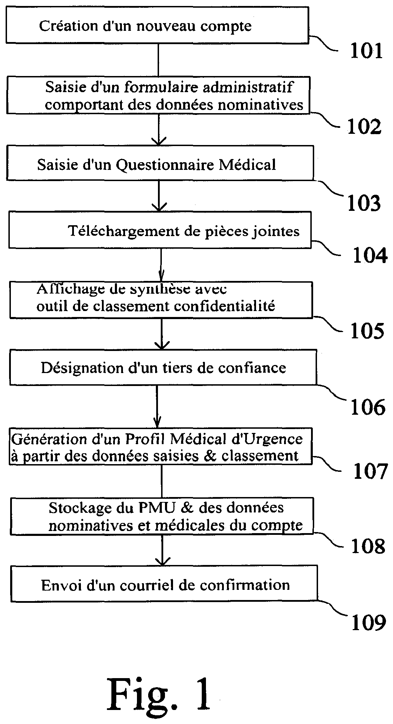

L'on décrit à présent, en référence à la figure 1, comment l'on peut mettre en place les éléments techniques permettant un accès sécurisé à un dossier médical numérique ou informatique stocké sur un serveur sécurisé distant, alors même que l'on se trouve dans une situation d'urgence, dans laquelle un patient inconscient, en état de choc ou tout simplement dans l'incapacité d'accéder lui-même à son dossier médical numérique, est pris en charge dans un service d'urgence d'un hôpital ou d'une clinique privée, voir dans tout cabinet médical susceptible d'accueillir une situation d'urgence.

D'une manière générale l'on considère un dossier médical numérique sous une forme quelconque et, plus spécifiquement, dans les modes de réalisation qui seront exposés ci-après, un ensemble de données médicales rassemblées notamment au moyen d'un Questionnaire Médical, spécialement conçu pour collecter de manière cohérente et classifiée diverses données médicales d'un utilisateur/patient potentiel.

Suivant un mode de réalisation, l'accès à un dossier médical contenant des données nominatives et confidentielles - lequel est stocké dans une zone protégée d'un serveur sécurisé distant - passe par la génération préalable - et automatique - d'un dossier médical d'urgence - ci-après désigné Profil Médical d'Urgence (PMU) - lequel est généré à partir des informations saisies par le patient lors de la constitution de son dossier médical comme cela sera décrit plus loin, et qui servira fonctionnellement à permettre un accès via un tiers de confiance.

### 1. Premier mode de réalisation : le PMU stocké sur un serveur sécurisé distant

Dans le premier mode de réalisation qui va être décrit à présent, le dossier médical numérique - mais également le Profil Médical d'Urgence (PMU) qui en est issu comme cela est décrit ci-après - ont vocation a être stockés sur un serveur distant sécurisé, destiné à permettre la dématérialisation des données et suivre leur titulaire au gré de ses déplacements.

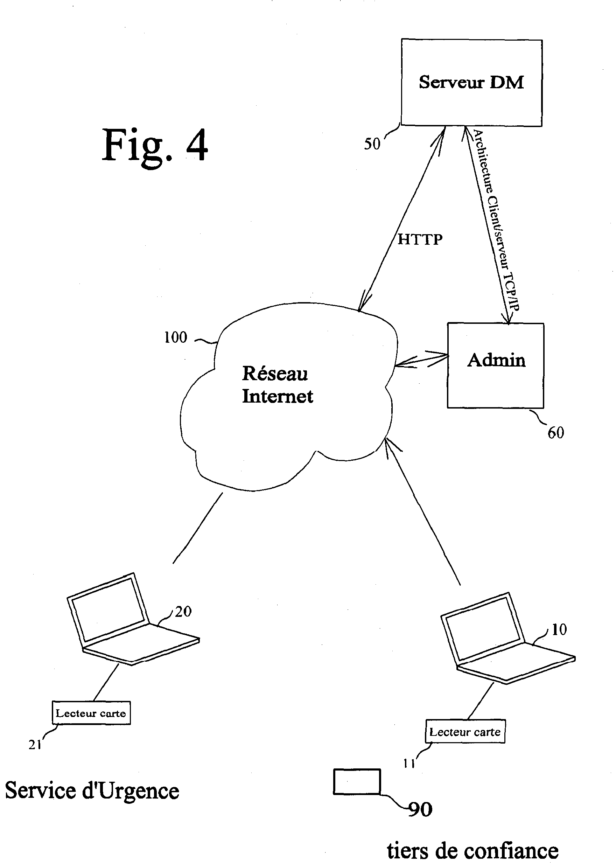

La figure 1 illustre plus particulièrement la procédure qui est mise en œuvre, notamment par le serveur sécurisé (illustré plus spécifiquement dans la figure 4 en relation avec la référence 50) pour créer un compte utilisateur, constituer le dossier médical et, par la suite, générer automatiquement le Profil Médical d'Urgence (PMU).

Dans une étape 101, un utilisateur disposant d'un moyen d'accès au réseau Internet, accède au serveur sécurisé et procède à la création d'un nouveau compte utilisateur. D'une manière concrète, cette création de compte comporte également la génération d'un identifiant et d'un mot de passe qui pourront être librement utilisés par le patient d'une manière conventionnelle pour un accès sans restriction à son dossier médical et notamment en vue de son examen et/ou de sa mise à jour. D'une manière générale, l'identifiant sera créé par le serveur sécurisé distant, soit de toute pièce, soit de manière secondaire à la suite de la présentation d'un autre identifiant primaire, tel que celui utilisé notamment pour l'accès aux services bancaires (Carte MasterCard, Diners, American Express - marques déposées par leurs titulaires respectifs).

Dans un mode de réalisation particulier, l'on pourra envisager qu'une telle création de compte passe par un abonnement, lequel pourra être ensuite matérialisé par la réception d'un support physique, tel que par exemple une carte médicale sous forme plastifiée affichant l'identifiant (mais non le mot de passe) destinée à demeurer dans le portefeuille du titulaire, en compagnie des autres de paiement/crédit etc...

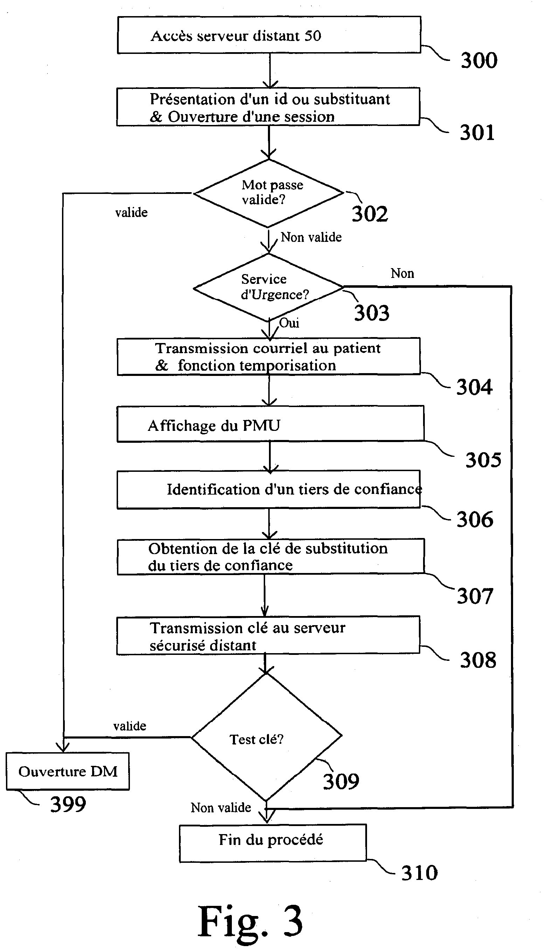

De préférence, le support physique se présentera sous la forme d'une carte, analogue à une carte de crédit/paiement, affichant de manière visible l'identifiant du compte ainsi qu'un QR code contenant l'adresse (U.R.L. *Uniform Resource Locator* dans la littérature anglo-saxonne) du serveur distant, ainsi qu'un ensemble de circuits électroniques associés à un connecteur USB, comme cela sera décrit en relation avec la figure 3. Ainsi, ce support physique permettra trois modes distincts d'accès au serveur sécurisé.

Dans une étape 102, le serveur génère un premier formulaire ou questionnaire de saisie d'informations d'ordre administratif, et notamment nominatives: tels que, par exemple: nom, date et lieu de naissance, adresse, téléphone, adresse courriel etc ...

Suivant un mode de réalisation particulier, et optionnel, le remplissage du formulaire administratif est réalisé au sein d'une interface graphique proposant un outil spécifique permettant à l'utilisateur d'attribuer une fonction d'identification partielle de substitution à un ou plusieurs des éléments nominatifs saisies, et dont l'ensemble, collectivement, permettra un accès au dossier médical suivant un mode d'urgence, selon les procédures d'accès qui seront décrites ci-après.

Puis dans une étape 103, le procédé procède, au moyen d'une interface graphique de saisie appropriée, à l'affichage d'un Questionnaire Médical se composant d'un ensemble de formulaires ou sous-questionnaires médicaux soigneusement élaborés et conçus de manière à permettre une collecte d'un grand nombre d'informations ou données médicales concernant le patient. La présentation du questionnaire pourra inclure des zones d'explication des questions, et fournir/présenter automatiquement des conseils adaptés en fonction de la réponse enregistrée.

Ces questionnaires peuvent être remplis soit par le patient lui-même, soit conjointement par le patient et son médecin, notamment lors d'une consultation qui pourra être une consultation dématérialisée telle que décrite dans la demande de brevet français 12/02401 en date du 10 Septembre 2012 intitulée « *Procédé d'accès et de partage d'un dossier médical* », et déposée par les demandeurs de la présente demande de brevet, qui présente l'avantage de permettre une validation immédiate par un professionnel de la Santé des données médicales ainsi saisies sur le serveur sécurisé. De manière plus générale, l'on pourra envisager l'utilisation des procédures décrites dans les demandes de brevet mentionnées précédemment, notamment pour permettre l'enrichissement du dossier médical stocké sur le serveur distant 50.

Lorsque c'est le patient /utilisateur qui instruit lui-même son dossier médical, le patient remplit lui-même les questionnaires qui lui sont soumis par l'interface du serveur sécurisé, en attendant que celui-ci puisse être validé par un praticien de son choix.
- Il pourra lui-même remplir les informations concernant sa santé en suivant un questionnaire précis, qui pourra s'afficher et s'enregistrer sous plusieurs langues.
- Ce questionnaire contiendra plusieurs catégories de questions destinées à intéresser les services d'urgences, mais il peut aussi contenir des questions plus spécifiques liées à des profils particuliers (sport, maladies chroniques, handicap, autonomie, psychisme, dépendance...)
- Ce questionnaire permettra tout type de question (questions à boulets, boutons radios, choix multiples, questions liées...) dont l'objectif de son organisation sera de formuler toute question d'une manière la plus intelligible et simple possible à son utilisateur afin d'obtenir les réponses les plus médicalement exactes et pertinentes.
- La synthèse de ce questionnaire aura pour objectif de présenter les informations saisies par le patient dans un ordre de priorité utile aux urgentistes (système de pondération qui affichera automatiquement les réponses dans un ordre souhaité par le corps médical, et non dans l'ordre saisi par le patient)

Dans un mode de réalisation particulier, l'interface graphique de saisie du questionnaire comporte un outil de téléchargement de pièces jointes, sous la forme d'une icône de forme quelconque, permettant de télécharger des fichiers électroniques de formats quelconques - notamment JPG, BITMAP, TIFF, PDF etc - depuis son ordinateur personnel vers le serveur de saisie, comme cela est illustré dans une étape 104 de la figure 1.

Il est à noter que ces pièces jointes représentatives de prescriptions, de certificats, de rapports d'analyse etc... présentent un grand intérêt dans la mesure où elles permettent de venir conforter une validation ultérieurement effectuée par le médecin du patient, voire même permettre dans l'action une validation par un médecin urgentiste ayant pris le patient inconscient en charge et qui ne serait pas le médecin réfèrent A cet égard, et cela correspond à un premier avantage du procédé décrit, les pièces jointes permettent d'apporter un premier niveau particulièrement significatif de validation médicale car ces pièces jointes seront, dans les conditions qui seront décrites ci-après, rendues accessibles à un médecin urgentiste même en cas de défaut du mot de passe associé à l'identifiant du compte, et ce grâce à la procédure de substitution qui sera décrite ci-après. Il est à noter que dans un mode de réalisation particulier, les pièces jointes pourront être stockées sur le même serveur sécurisé destiné à stocker le dossier médical, voire sur un serveur distinct.

Lorsque le Questionnaire Médical est complètement rempli par l'utilisateur, le procédé procède ensuite à l'affichage d'un écran ou plusieurs écrans de synthèse, dans une étape 105, venant résumer les différents éléments d'information composant les différents sous-questionnaires constitutif du Questionnaire Médical.

Dans un mode de réalisation, l'interface graphique proposé au titulaire, lors de l'affichage des écrans de synthèse, propose un outil de classification lui permettant d'affecter ou non un caractère confidentiel à chacun des éléments constitutifs du Questionnaire Médical par l'attribution d'un champ spécifique caractéristique du caractère confidentiel. Cette fonctionnalité est, comme on va le voir, particulièrement importante puisqu'elle a vocation à confier au patient le libre arbitre en ce qui concerne le caractère confidentiel ou non qu'il convient d'attribuer à chacun des éléments d'information constituant son *propre* Dossier Médical et, par suite, de permettre une génération automatique et circonstanciée du Profil Médical d'Urgence (PMU), qui pourra servir de premier lien entre le médecin urgentiste et son patient inconscient et, par suite, de vecteur d'accès au dossier médical complet de ce dernier.

Dans un mode de réalisation particulier, le procédé ne valide le Questionnaire Médical (et par conséquent tous les sous-questionnaires le composant) qu'après l'achèvement complet de la classification opérée par le patient.
- Le patient aura donc décidé de publier ou non chacune des informations contenue dans la synthèse (considération faite de ce que l'article 8 de la Loi Informatique et Liberté permet à toute personne de rendre publiques, les informations qui le concernent) qui s'enregistreront sous la forme d'un document accessible sur le serveur sécurisé et agréé.
- Ce document pourra être enregistré sur le port USB de la carte.

Puis, dans une étape 106, le serveur sécurisé sollicite du titulaire du compte nouvellement crée la désignation d'au moins un tiers de confiance, avec la saisie d'éléments d'identification tel que le nom, les coordonnées téléphoniques, l'adresse courriel etc... Dans un mode de réalisation particulier, le titulaire du compte se voit offrir la possibilité de désigner plusieurs tiers de confiance (au minimum 3). Dans un mode de réalisation particulier, ce tiers ou ces tiers de confiance se verront formellement attribuer une clé de substitution, par exemple sur un support physique telle qu'une carte, laquelle sera également enregistrée dans une table stockée dans la zone protégée du dossier médical du patient. Dans un mode de réalisation particulier, la clé de substitution pourra être tout simplement le propre identifiant du tiers de confiance pour son propre Dossier Médical.

Dans un autre mode de réalisation, la clé de substitution pourra être réduite à sa plus simple expression, et sera remplacée par tout autre moyen technique (mail, sms, icône d'une application, smartphone...) permettant au tiers de manifester sa volonté de permettre au médecin urgentiste d'accéder au dossier du patient inanimé et de valider auprès du serveur sécurisé la demande d'ouverture du dossier médical confidentiel et/ou nominatif. Cette validation déclenchera automatiquement l'envoi d'un mot de passe aléatoire et temporaire au serveur/ordinateur du praticien ou du service d'urgence demandeur.

Il est à noter, et cela est un élément important, que la clé de substitution, lorsque celle-ci lui a été formellement attribuée ne se confond par avec le mot de passe qui est régulièrement attribué au titulaire du compte et propriétaire de son dossier médical. En effet, l'on peut imaginer que le lien de confiance entre un titulaire et son tiers de confiance soit suffisamment fort pour que les deux partagent le mot de passe permettant ainsi à l'un et à l'autre d'accéder librement, sans restriction particulière, au dossier médical. En revanche, lorsque le tiers de confiance ne détient qu'une clé de substitution - laquelle pourra être formalisée par un numéro d'identification ou même de manière virtuelle par une donnée dans un téléphone mobile comme on le verra ci-après - cette clé de substitution ne peut servir à constituer un second accès « parallèle » au dossier médical. Elle n'a vocation à servir que dans le cadre d'une procédure d'urgence comme cela sera décrit ci-après.

A l'issue de la déclaration du tiers de confiance lors de l'étape 106, le serveur procède alors, dans une étape 107 - et il s'agit d'un élément particulièrement avantageux - à la génération automatique d'un Profil Médical d'Urgence (PMU) comportant des informations vitales, quoique non nominatives et non confidentielles, et judicieusement présentées dans leur ordre d'importance et de pertinence pour les besoins d'un service d'urgence. Optionnellement, une interface graphique permettra la mise en œuvre d'un outil permettant de modifier l'ordre de priorité de présentation des informations du Profil Médical d'Urgence (PMU). De préférence, le PMU sera multi-lingue de manière à pouvoir être automatiquement affiché dans une langue d'accueil déterminée. Clairement, il ne s'agit que d'exemples non limitatifs.

Dans des modes de réalisation spécifiques, le procédé permet également la génération d'autres types de profils médicaux plus spécifiquement adaptés à certaines catégories de patients/utilisateurs, et notamment :
- un Profil Médical Handicap comportant un abstract judicieusement organisé d'informations médicales -non nominatives et jugées non confidentielles par le titulaire - organisées pour correspondre aux exigences d'un mode de vie soumis à un handicap ;
- un Profil Médical Sport-Santé comportant un abstract judicieusement organisé d'informations médicales - non nominatives et jugées non confidentielles par le titulaire - organisé pour correspondre aux exigences de mode de vie d'un sportif de bon niveau ;
- un Profil Médical Chronique comportant un abstract judicieusement organisé d'informations médicales - non nominatives et jugées non confidentielles par le titulaire - organisé pour répondre aux besoins liés à des maladies d'ordre chronique ;
- un Profil Médical « baby », enfant, sénior, etc...

Ces divers Profils Médicaux, et notamment le Profil Médical d'Urgence (PMU) sont générés automatiquement par le serveur, en fonction des informations collectées lors du remplissage des formulaires (et ultérieurement validées par le médecin), mais également en fonction des diverses classifications opérées par le titulaire du compte.

Ainsi, s'il est bâti sur les fondations des données médicales saisies par le patient et ultérieurement validées par un médecin, le Profil Médical d'Urgence (PMU) est automatiquement généré par le procédé et essentiellement déterminé par le patient qui, au cas par cas, aura attribué un attribut de confidentialité à chacune des données saisies lors de l'étape 105.

De ce fait, le Profil Médical d'Urgence (PMU) se distingue fondamentalement du dossier médical numérique, tant dans sa structure interne que dans sa fonction.

D'abord, s'agissant de sa structure, le Profil Médical d'Urgence (PMU) est un ensemble de données, généré automatiquement suivant le procédé de la figure 1, et qui ne comporte que des informations **NON NOMINATIVES** et classifiées **NON CONFIDENTIELLES**, ainsi qu'une évocation - non confidentielle - d'informations confidentielles susceptibles de se trouver dans le dossier médical stockées dans le serveur sécurisé. Par conséquent, si ce dernier est voué à s'enrichir continuellement, au rythme des aléas et des interventions qui ponctuent la vie du patient, le Profil Médical d'Urgence (PMU) a - lui - une vocation à une certaine stabilité puisqu'il constitue un résumé, un abstract, et recense les données les plus importantes - non nominatives et non confidentielles - pour un service d'urgence susceptible de prendre un jour en charge le patient.

Par ailleurs, s'agissant de sa fonction, l'on va constater, avec les procédures qui sont décrites ci-après, que le Profil Médical d'Urgence (PMU) sert de clé, de vecteur d'accès au dossier médical dans le cadre d'une procédure d'urgence. A cet effet, le PMU permet un accès direct au serveur sécurisé dans le but de permettre l'ouverture complète du dossier médical du patient, notamment via le recours à l'un des tiers de confiance préalablement désignés par le titulaire du dossier médical. Dans un mode de réalisation particulier, le Profil Médical d'Urgence (PMU) comporte un identifiant ou un lien d'accès non nominatif vers le ou les tiers de confiance désigné(s) par le titulaire.

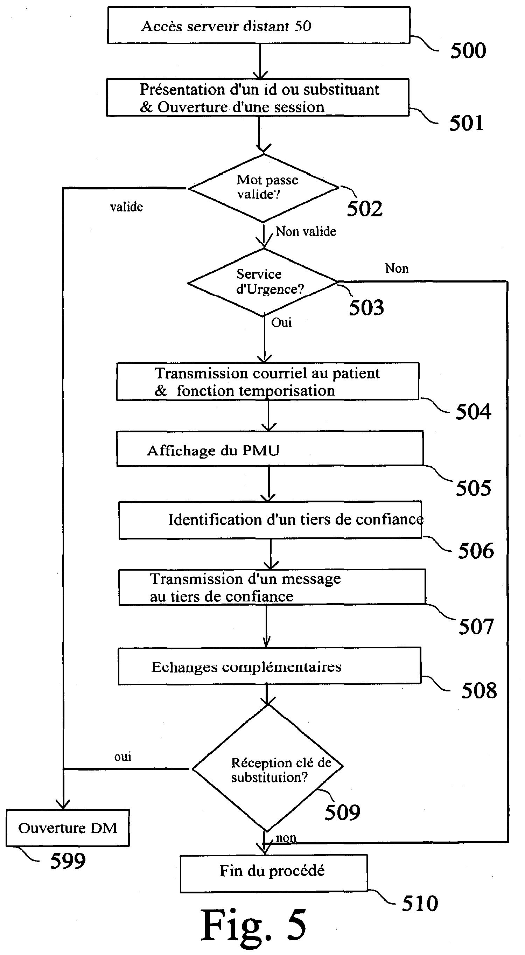

En revenant à nouveau au procédé de la figure 1, l'on voit que, à la suite de la génération du Profil Médical d'Urgence (PMU) de l'étape 107, le procédé procède, dans une étape 108, au stockage de ce dernier, de préférence sur le serveur sécurisé (mais également sur le support physique dans le cas de la carte médicale de la figure 5), en même temps que la totalité des informations médicales et administratives qui ont été saisies par le titulaire du compte, et notamment les clés de substitutions potentiellement allouées au(x) tiers de confiance. Il est à noter que toutes les informations nominatives et/ou confidentielles sont stockées dans une zone protégée du serveur sécurisé, laquelle n'est accessible en principe qu'au moyen de la présentation de l'identifiant du titulaire du compte **avec** son mot de passe associé ou, alternativement, avec une clé de substitution suivant les procédures d'accès d'urgence qui seront décrites plus loin.

Au terme de la procédure de création de compte et d'enregistrement du Questionnaire Médical, le serveur sécurisé transmet dans une étape 109 un courriel de confirmation au titulaire du compte.

Il est à noter que le titulaire du compte, détenant l'identifiant celui-ci ainsi que le mot de passe y associé, pourra bien évidemment accéder à nouveau ultérieurement à son compte de manière à mettre à jour son dossier médical en venant y intégrer de nouvelles données et/ou pièces jointes. Il pourra également, modifier la ou les désignations des tiers de confiance en tant que de besoin, et les mises à jour ainsi faites seront clairement répercutées, lorsque cela sera nécessaire (notamment en ce qui concerne la désignation des tiers de confiance) au niveau du Profil Médical d'Urgence.

Comme on le voit, le procédé décrit précédemment aboutit à la génération, d'une manière complètement automatique, d'un Profil Médical d'Urgence (PMU) ne comportant que des informations non nominatives et jugées non confidentielles (car classées comme telles) par le titulaire du compte - et qui serviront fonctionnellement à l'accès au dossier médical numérique.

Il est important de noter - et cela est un avantage considérable de la présente invention - qu'il n'est nullement nécessaire de présenter le mot de passe associé à l'identifiant pour prendre connaissance du Profil Médical d'Urgence (PMU). En effet, celui-ci sera accessible dès lors que l'on détiendra le numéro d'identification du titulaire de la carte ou plus généralement lorsque l'on sera en possession de la carte attribuée au titulaire du compte ou, encore, lorsque l'on présenter un ensemble d'éléments d'identification suffisants, correspondant à la liste crée par le patient dans l'étape 102.

Ainsi, dans une situation d'urgence, comme on le verra plus en détail ci-après, un médecin urgentiste qui aura à prendre en charge un patient inanimé ou en état de choc, aura la possibilité d'accéder directement à son Profil Médical d'Urgence (PMU), simplement en se saisissant de sa carte détenue dans son portefeuille, en utilisant le numéro de la carte.

Dans un mode de réalisation particulier, la carte comporte un QR code permettant, grâce à un logiciel de lecture de code QR approprié, d'accéder directement sur le site du serveur sécurisé, et même de provoquer le téléchargement du fichier PDF stockant le Profil Médical d'Urgence (PMU). De cette manière, le médecin urgentiste peut immédiatement prendre connaissance des informations de survie essentielles que contient Profil Médical d'Urgence (PMU), et notamment des signes distinctifs généraux - taille, poids, couleur des cheveux, cicatrices etc... - permettant de conforter l'idée que la carte que détient le médecin urgentiste appartient réellement à la personne qu'il est amené à prendre en charge. Le médecin urgentiste peut ainsi obtenir immédiatement, alors même que le patient qui est en état de choc ou inconscient et n'est pas en mesure de présenter son identifiant et son mot de passe, une première série d'informations (groupe sanguin, allergies etc...) qui peuvent s'avérer particulièrement précieuses pour les traitements et interventions qui devront être envisagés.

Ces informations peuvent être obtenues immédiatement et en cela, l'invention apporte un avantage décisif. Par ailleurs, comme ni la carte ni le Profil Médical d'Urgence (PMU) ne comporte d'informations nominatives ou jugées confidentielles par le titulaire de la carte, la perte ou le vol de la carte restera sans grande conséquence.

Il peut s'avérer cependant critique pour le médecin urgentiste de prendre connaissance de données médicales plus approfondies et qui ne figurent pas dans le Profil Médical d'Urgence (PMU) mais dont la présence y sera évoquée ou mentionnée. Ou encore, il pourra être utile, dans des circonstances particulières, que le médecin urgentiste effectue un examen plus approfondi du contenu du dossier médical, y compris les données les plus confidentielles, et notamment telle ou telle annexe ou pièce jointe venant corroborer une déclaration particulière, dans le but de confirmer un diagnostic ou d'affiner la pertinence d'un traitement à envisager.

Bien évidemment, le médecin urgentiste souhaitant accéder à de telles information nominatives et/ou confidentielles, ou tout simplement aux annexes et pièces jointes, sera requis par le serveur sécurisé 50 de présenter, outre l'identifiant déjà présenté, le mot de passe correspondant au compte du titulaire que, en règle générale, il ne possède nullement. Afin de répondre à ce besoin critique, le procédé que l'on va à présent décrire met en place, en combinaison avec le concept de Profil Médical d'Urgence (PMU), celui de tiers de confiance permettant d'obtenir une clé de substitution pouvant servir dans le cadre d'une procédure d'urgence pour accéder au dossier médical complet du patient, incluant notamment les données nominatives et/ou confidentielles que celui-ci peut comporter.

A cet effet, l'on dispose un lien d'accès vers au moins un tiers de confiance au sein du Profil Médical d'Urgence (PMU), de manière à permettre au médecin urgentiste d'obtenir une clé de substitution. Sous la forme la plus immédiate, le lien d'accès pourra endosser la forme d'un numéro de téléphone inscrit dans le PMU, numéro que le médecin pourra appeler pour tenter de joindre le tiers de confiance et obtenir ainsi une clé de substitution qui pourra être reconnue par le serveur sécurisé comme constituant une alternative au mot de passe manquant ou défaillant. Alternativement, comme cela sera décrit plus loin, c'est la réaction elle-même du tiers de confiance, à savoir sa manifestation de volonté qui pourra prendre diverses formes (envoi d'un SMS, d'un courriel, appel d'un serveur vocal etc...) qui constituera la clé de substitution mise en oeuvre dans la procédure d'accès au serveur sécurisé.

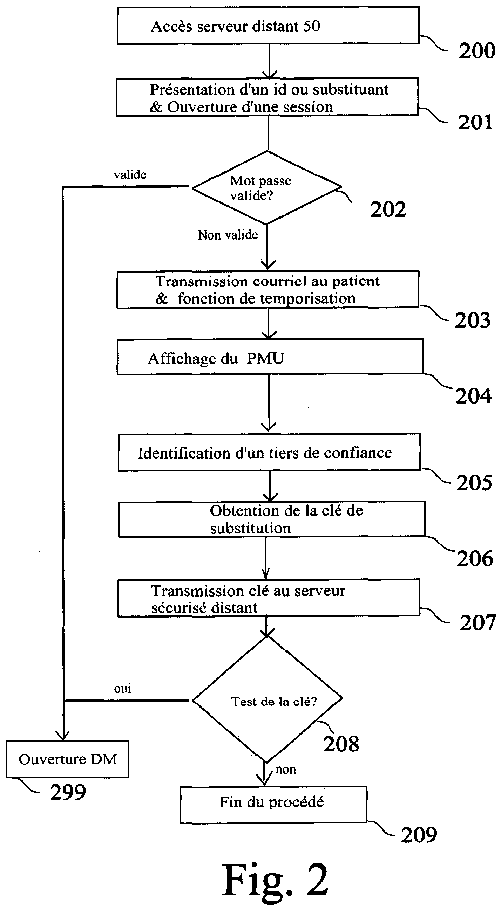

La figure 2 illustre plus particulièrement les étapes du procédé permettant d'accéder, dans un premier temps, au Profil Médical d'Urgence (PMU) stocké sur le serveur sécurisé distant 50 pour, dans un second temps, accéder au dossier médical nominatif et confidentiel.

Dans une étape 200, l'on considère une situation dans laquelle un médecin urgentiste est appelé à prendre en charge un patient en état de choc ou même inanimé. Il ne détient que le support physique, attestant que son patient est vraisemblablement titulaire d'un dossier médical, et en particulier un dossier médical d'urgence PMU stocké sur un serveur sécurisé. Le médecin procède, grâce au support détenu, à un accès au serveur distant sécurisé 50 (illustré en figure 4).

De manière concrète, cet accès au serveur sécurisé pourra revêtir diverses formes. L'on pourra en premier lieu accéder directement depuis un navigateur au serveur via son adresse générique. En second lieu, il sera possible de scanner le QR code présent sur la carte médicale du patient, lequel QR code comportera l'adresse URL de serveur. Enfin, et cela est une troisième possibilité, le médecin urgentiste pourra accéder directement au serveur sécurisé distant en venant brancher le connecteur USB de la carte de la figure 5 sur son ordinateur, ce qui entraînera automatiquement un accès au serveur sécurisé.

Puis, dans une étape 201, le procédé procède à l'ouverture d'une session entre le système de traitement de données du médecin urgentiste (représenté par le dispositif 20 de la figure 4) et le serveur distant DM, suite à la présentation de l'identifiant du compte affiché sur la carte médicale du patient.

Alternativement, et ceci est une option possible, le serveur sécurisé pourra accepter, au lieu et à la place de l'identifiant régulièrement attribué au compte - et affiché sur la carte médicale - un jeu d'identifiants de substitution tels que définis lors du remplissage du formulaire administratif de l'étape 102. De cette manière, lorsque le patient ne détient pas, dans son portefeuille, sa carte médicale comportant l'identifiant requis, le médecin urgentiste peut tenter d'obtenir, dès lors qu'il obtient suffisamment d'informations nominatives concernant le patient, un accès de substitution au serveur sécurisé. Cela sera par exemple le cas lorsque le médecin urgentiste sera en présence d'une carte nationale d'identité, d'un passeport, d'un permis de conduire etc..., autant d'éléments susceptibles de permettre le remplissage des champs correspondants aux informations nominatives définies comme étant des identifiants de substitutions potentielles... susceptible d'autoriser l'ouverture d'une session avec le serveur sécurisé 50.

D'une manière générale, l'ouverture d'une session est bien connue d'un homme du métier et pourra se formaliser via un navigateur quelconque, tel qu'INTERNET EXPLORER de la société MICROSOFT Corp., au moyen de requêtes HTTPS (*Hyper Text Transport Protocol*) sécurisées.. Il n'est clairement pas nécessaire, dans un souci de concision, de détailler plus avant les procédures à mettre en oeuvre à cet effet.

Il est à noter toutefois, que lors de l'établissement de cette session qui s'ouvre à l'initiative du système 20 du médecin urgentiste, diverses procédures de vérification et pourront être fort opportunément mises en oeuvre par le serveur sécurisé 50, notamment la vérification et/ou l'enregistrement de l'adresse IP etc... de manière à permettre la traçabilité et à faciliter la constitution d'un dossier de plainte pénale, notamment en cas de fraude avérée. De manière plus générale encore, l'on pourra envisager que le système 20 du médecin urgentiste aura fait préalablement l'objet d'une procédure d'authentification forte auprès du serveur sécurisé 50, notamment au moyen d'un enregistrement préalable de paramètres systèmes (tel que numéro de série de disque dur, présence de certains périphériques, une carte CPS si elle existe etc...), lesquels pourront avantageusement permettre un référencement systématique par le serveur sécurisé et, finalement, une authentification et une sécurité accrue de l'accès au dossier médical du patient.

Dans une étape 202, le serveur sécurisé vérifie la présence d'un mot de passe ou la validité d'un mot de passe saisi.

Dans l'hypothèse où un tel mot de passe valide est établi - ce qui correspond par exemple à la situation régulière d'un patient pouvant communiquer avec l'urgentiste et lui transmettre le mot de passe de son Dossier Médical - alors le procédé poursuit par une étape 299 permettant l'ouverture du Dossier Médical complet, à savoir incluant les informations nominatives mais également confidentielles.

Au contraire, en cas de défaut de mot de passe valide, le procédé poursuit avec une étape optionnelle 203, le procédé procède à l'envoi par le serveur sécurisé 50 d'un courriel et/ou message SMS à destination du titulaire de la carte, éventuellement avec un ensemble d'informations collectées lors de la vérification effectuées dans l'étape 201, puis met en œuvre une fonction de temporisation prédéfinie, par exemple de 5 ou 10 minutes. Cette étape 202 permet ainsi de sécuriser potentiellement l'accès au dossier médical numérique dans la mesure où, dans le cas d'un accès frauduleux, le titulaire de la carte sera alors en mesure, durant le délai de temporisation qui lui est offert, de s'opposer à l'ouverture de son dossier médical.

L'on notera, à cet effet, que lors de l'enregistrement du patient et de la création de son compte, ce dernier pourra être invité à télécharger, depuis le site du serveur distant, une application sur son ordinateur, sur sa plaquette tactile ou sur son téléphone mobile intelligent (*smartphone*), de manière à permettre la gestion des messages susceptibles d'être reçus du serveur sécurisé. Alternativement, le message de l'étape 203 pourra prendre la forme d'un message SMS ou d'un courriel comportant un lien URL permettant au patient de venir directement bloquer l'accès à son dossier médical numérique.

Puis, dans une étape 204, le procédé procède à l'affichage, sur le système 20 du médecin urgentiste du Profil Médical d'Urgence (PMU) stocké sur le serveur distant (50).

Dans un mode de réalisation particulier, la vérification de l'adresse IP effectuée par le serveur sécurisé procède à l'ouverture du Profil Médical d'Urgence (PMU) dans la langue du pays correspondant à l'adresse IP répertorié, de manière à faciliter la lecture du dossier dans le cas où le patient est pris en charge par un service d'urgence situé à l'étranger.

Dans une étape 205, le procédé tente une identification d'un tiers de confiance dans le but d'obtenir une information d'identification qui pourra être acceptée par le serveur sécurisé

De la manière la plus simple, on pourra prévoir que le système affiche un numéro de téléphone mobile d'un ou plusieurs tiers de confiance, de manière à permettre la récupération, dans une étape 206, d'une clé de substitution pouvant se substituer au mot de passe non connu

Puis, dans une étape 207, le système 20 du médecin urgentiste transmet au serveur sécurisé la clé de substitution obtenue du tiers de confiance.

Dans une étape 208, le serveur sécurisé distant 50 procède à la vérification de la clé de substitution.

Si la clé de substitution est répertoriée dans la table de correspondance comme étant valide alors le serveur 50 autorise l'ouverture du dossier médical , dans une étape 299.

Au contraire, si la clé de substitution n'est pas reconnue comme étant valide par le serveur 50, alors le procédé s'arrête dans une étape 209 sans que les informations nominatives et/ou confidentielles - et notamment les pièces jointes - aient pu être affichées. De manière complémentaire, un message récapitulatif pourra être transmis par courriel au titulaire de la carte pour résumer la séquence des messages échangés.

Dans un autre mode de réalisation simplifié, on peut prévoir que le tiers de confiance (ayant égaré/oublié sa clé de substitution) puisse par tout autre moyen technique (sms, email, application smartphone ou autre) envoyé une information spécifique au serveur sécurisée susceptible d'être reconnue comme « clé de substitution » déclenchant l'autorisation d'ouverture du dossier confidentiel et/ou nominatif.

Plus directement encore, l'on pourra considérer de manière très générale, comme étant une clé de substitution utilisable pour les procédures décrites dans la présente demande de brevet, toute manifestation de la volonté de l'un ou plusieurs des tiers de confiances désignés par le titulaire du dossier. De préférence, cette manifestation de volonté pourra prendre la forme d'un SMS (en réponse à un SMS transmis par le serveur sécurisé), un courriel (en réponse à un courriel transmis par le serveur sécurisé) venant formaliser l'accord du tiers de confiance concerné pour la procédure d'ouverture d'urgence du dossier médical. Dans un mode de réalisation particulier, l'on pourra même envisager une clé de substitution sous la forme d'un appel vocal transmis depuis le téléphone mobile attribué au tiers de confiance, lequel appel vocal sera enregistrer dans le but d'une traçabilité des caractéristiques vocales du tiers de confiance ayant autorisé l'accès au dossier médical.

Dans un mode de réalisation spécifique, la manifestation de volonté pourra être sécurisé par l'utilisation d'une application spécifique présente sur un téléphone mobile du tiers de confiance, et permettant une gestion sécurisée (plus sécurisée que ne serait l'envoi d'un SMS) d'une autorisation transmise au serveur distant.

Il est à noter que toute validation/autorisation confirmée en provenance d'un tiers de confiance déclenchera l'ouverture du dossier médical confidentiel et/ou nominatif auprès du praticien/service demandeur.

L'ouverture du dossier pourra être automatisée si les serveurs respectifs sont restés ouverts et connectés, ou s'ils sont en capacité de correspondre par des logiciels/exécutables connectés et en lien respectifs.

Sinon, cette validation déclenchera automatiquement depuis le serveur sécurisé, l'envoi d'un mot de passe aléatoire et temporaire au serveur/ordinateur du praticien ou du service d'urgence demandeur.

### 2. Second mode de réalisation : accès restreint avec authentification du service d'urgence

Le premier mode de réalisation qui vient d'être décrit montre comment l'on peut, en passant par l'affichage du Profil Médical d'Urgence et l'obtention éventuelle d'une clé de substitution détenue par un tiers de confiance ou alternativement d'une manifestation de volonté de ce dernier, de procéder à l'accès du dossier médical du titulaire, alors même que l'on ne dispose pas du mot de passe associé à l'identifiant du compte.

De ce fait, il en résulte que, théoriquement, le tiers de confiance peut être amené à consulter fréquemment le dossier médical du titulaire, alors même qu'il ne détient pas explicitement le mot de passe, ce qui peut ne pas être souhaitable.

Cette situation est susceptible de créer une faille de sécurité qui peut être préjudiciable et le procédé de la figure 3 illustre un second mode de réalisation, plus sophistiqué, permettant d'éviter cet inconvénient. En effet, dans ce second mode de réalisation, la clé de substitution *détenue* par le tiers de confiance ou sa manifestation de volonté n'est pas utilisable pour un accès direct au dossier médical, mais ne peut servir que dans le cas d'une réelle procédure d'urgence vérifiée par le serveur sécurisée.

Les étapes 300-301 et 302 sont identiques aux étapes 200-201-202 de la figure 2.

Dans une étape 300, le procédé effectue un accès au serveur sécurisé 50.

Dans une étape 301, l'on procède à la présentation de l'identifiant du compte (numéro de la carte médicale) ou, à défaut, des éléments d'identification de substitution préalablement définis par le titulaire du compte lors de l'étape 102, permettant ainsi l'ouverture d'une session.

Dans une étape 302, le procédé teste la présence d'un mot de passe valide et, dans l'hypothèse d'un tel mot de passe, permet l'ouverture du dossier médical dans une étape 399.

Au contraire, en cas de défaut de mot de passe, le procédé poursuit alors par une étape de vérification 303 d'une situation d'urgence. Dans un mode de réalisation particulier, cette vérification passe par la récupération de l'adresse IP du système 20, ainsi que par l'obtention de toute une série d'informations diverses susceptible de valider de confirmer que la demande d'accès est bien présentée dans le cadre d'une procédure d'urgence. En particulier, l'on peut envisager une procédure d'enregistrement auprès du serveur sécurisé du service d'urgence ou la vérification de la Carte Professionnelle de Santé du médecin urgentiste, de manière à valider l'étape de vérification 303. Alternativement, l'on peut également organiser un référencement systématique des systèmes utilisés dans les services d'urgences utilisant fréquemment un accès au serveur sécurisé 50, notamment en procédant à une inscription et un enregistrement systématiquement des éléments matériels de ces mêmes systèmes.

D'une manière générale, tout système d'authentification forte pourra être fort opportunément utilisé.

Si le test de l'étape 303 n'aboutit pas, alors le procédé poursuit immédiatement avec une étape terminale 310 mettant un terme à la procédure d'accès au dossier médical. Optionnellement, le procédé pourra transmettre un message de synthèse au titulaire du compte l'informant de ce qu'un accès avorté s'est produit sur son dossier.

Si, au contraire, le test de l'étape 304 aboutit, alors le procédé poursuit avec les étapes 304-310 qui sont respectivement identiques aux étapes 203-209 de la figure 2.

En particulier :
Dans une étape optionnelle 304, le procédé procède à l'envoi par le serveur sécurisé 50 d'un courriel et/ou message SMS à destination du titulaire de la carte, éventuellement avec un certain nombre d'informations collectées lors des vérifications effectuées dans les étapes 301 et 303, puis met en œuvre une fonction de temporisation prédéfinie.

Puis, dans une étape 305, le procédé procède à l'affichage, sur le système 20 du médecin urgentiste du Profil Médical d'Urgence (PMU) stocké sur le serveur distant (50), éventuellement dans la langue d'accueil du service d'urgence.

Dans une étape 306, le procédé tente une identification d'un tiers de confiance dans le but d'obtenir une information d'identification qui pourra être acceptée par le serveur sécurisé

Dans une étape 307, la clé de substitution est récupérée et, finalement, présentée au serveur sécurisé dans une étape 308.

Dans une étape 309, le serveur sécurisé distant 50 procède à la vérification de la clé de substitution et, en cas de succès de cette vérification, le serveur 50 autorise l'ouverture du dossier médical, dans une étape 399.

Comme précédemment, l'on pourra également considérer comme étant une clé de substitution toute manifestation de volonté formalisée par l'un des tiers de confiance, et notamment l'envoi d'un SMS (en réponse à un SMS transmis par le serveur sécurisé), d'un courriel (en réponse à un courriel transmis par le serveur sécurisé) ou un appel vocal, etc...

Les procédures qui viennent d'être exposées présentent un avantage significatif, en ce qu'elles autorisent une automatisation très poussée permettant d'envisager de nouvelles possibilités d'accéder de manière très performante aux dossiers médicaux dématérialisés des patients.

En effet, il convient de souligner que dans la situation qui est considérée, le temps est vital et le tiers de confiance n'est nullement un professionnel susceptible d'agir avec sérénité dans une situation de crise. Le jour venu, il faudra qu'il intervienne avec sa sensibilité - et ses faiblesses - pour suppléer au défaut de mot de passe en raison de l'état de choc du titulaire de la carte. De la sorte, le tiers de confiance risque lui également de se trouver dans un état de choc important et il convient alors d'organiser un système d'accès très sécurisé au dossier médical du titulaire de la carte, et ce notamment en permettant d'éviter les erreurs dans les numéros d'identification etc...

C'est le but des trois modes de réalisations que l'on va décrire à présent, qui permettent d'automatiser pour une grande part la procédure d'accès, de manière à éviter les erreurs qui pourraient s'avérer fatales pour le titulaire du dossier médical, tout en autorisant une grande traçabilité des échanges - sous la gestion du serveur sécurisé - de sorte qu'il sera possible de revenir, ultérieurement et en tant que de besoin, sur le détail des étapes qui se sont déroulées lors de la procédure d'accès.

Les trois modes de réalisation supplémentaires qui vont être décrit à présent, et qui portent sur des formes plus sophistiquées et plus automatisées permettent d'obtenir la communication d'une clé de substitution valide, que la procédure soit centralisée autour du serveur sécurisé distant (3^{ème} mode de réalisation) ou directement par le système du médecin urgentiste (5^{ème} mode de réalisation). L'on décrira également, dans un 4^{ème} mode de réalisation l'utilisation d'un support physique sophistiqué, sous la forme d'une carte médicale dotée d'une électronique propre permettant le stockage direct du Dossier Médical d'Urgence (DMU).

### 3. Troisième mode de réalisation : procédures automatiques d'obtention de la clé de substitution centralisées par le serveur sécurisé distant

Pour illustrer ce troisième mode de réalisation, on se reportera plus spécifiquement au schéma de la figure 4, lequel illustre l'accès au réseau sécurisé distant 50 via un réseau Intranet ou le réseau Internet, lequel pourra centraliser toute la procédure d'obtention de la clé de substitution en vue d'un accès au dossier médical nominatif et confidentiel.

Le service d'urgence est doté d'un système de traitement de données, représenté sur la figure par un ordinateur portable 20, disposant d'un accès au réseau Internet 100 et par son biais au serveur sécurisé distant DM 50.

Le système 20 du service d'urgence est en outre associé à un dispositif d'authentification, de type lecteur de carte 21, lequel pourra servir à des fins diverses, et notamment par exemple pour le branchement d'une carte d'authentification permettant d'assurer l'authentification des requêtes émisses par le système 20. L'on pourra par exemple envisager un lecteur destiné à recevoir la Carte de Professionnel de Santé (CPS) qui est en usage en France, ou tout système d'authentification forte basée sur l'usage d'une carte à puce... Alternativement, l'on pourra envisager pour le praticien un système d'authentification basée sur une clé USB propre au praticien, voire un système autonome, comme un téléphone mobile intelligent (*smartphone* suivant la terminologie anglo-saxonne) doté de moyens de moyens d'authentification, telle que signature électronique, clés de cryptage.

L'on considère également un tiers de confiance qui dispose, de son côté, de son propre système de traitement de l'information 10, représenté par exemple par un ordinateur portable accédant au réseau Internet 100 et de son propre lecteur de carte 11. Alternativement, il pourra s'agir d'un système de type téléphone mobile intelligent (*smartphone*), un assistant personnel portable (PDA), une tablette tactile etc... et disposant eux également d'un accès au réseau Internet.

L'on a représenté sur la figure 4, à des fins d'illustration, un serveur administrateur 60 optionnel chargé de l'administration et de la gestion des dossiers médicaux, notamment des inscriptions et de la facturation.

L'on supposera en outre que les systèmes 10 et 20 sont dotés de moyens de notifications, de type courrier électronique, ainsi que d'un navigateur, comme Internet Explorer de l'éditeur MICROSOFT Corp. par exemple, permettant d'accéder simplement, via le protocole standardisé H.T.T.P. (*Hyper Text Transfer Protocol*) au serveur DM 50. De tels moyens sont bien connus d'un homme du métier et ne requièrent pas de développement supplémentaire. L'on pourra également envisager l'utilisation de logiciels et programmes plus spécifiques pour la mise en oeuvre des notifications et des procédures décrites ci-après. Par ailleurs, on pourra également envisager que les notifications transmises via l'un des systèmes 10, ou 20, résultent d'une notification transmise via un serveur extérieur.

La figure 5 illustre plus particulièrement les étapes du procédé permettant d'automatiser l'accès au dossier médical nominatif et confidentiel, et ce au moyen d'une procédure centralisée au niveau du serveur sécurisé distant DM 50.

D'une manière générale, les étapes 500- 503 illustrées dans la figure 5 sont similaires aux étapes 300-303 de la figure 3.

Ainsi, dans une étape 500, le système 20 du médecin urgentiste procède à un accès direct au serveur sécurisé 50, notamment via le navigateur présent dans le système d'exploitation.

Puis, dans une étape 501, le procédé procède à la présentation de l'identifiant du compte ou, alternativement de la série d'identifiant de substitution définis par le titulaire dans l'étape 120, en vue de l'ouverture d'une session entre le système 20 et le serveur sécurisé 50.

Puis, dans une étape 502, le procédé teste la présence d'un mot de passe valide, que le médecin urgentiste aura pu obtenir, par exemple, du titulaire du compte lui-même. Si le mot de passe est reconnu comme étant valide, alors le procédé poursuit avec une étape 599 permettant l'accès complet au dossier médical.

Au contraire, en cas de défaut de mot de passe, le procédé poursuit par une étape 503 qui correspond à une vérification d'une situation d'urgence. A cet égard, comme précédemment, il pourra être particulièrement opportun de prévoir, dans un souci de renforcement de l'authentification du système 20, diverses procédures permettant l'authentification forte du système 20, et la traçabilité des échanges entre celui-ci et le serveur. L'on pourra ainsi recourir fort opportunément aux procédures d'enregistrement préalable, aux procédés de signature électroniques ainsi qu'aux procédures d'authentification fortes basées notamment sur la reconnaissance de caractéristiques matérielles du système.

Puis, dans une étape optionnelle 504, le procédé procède à la transmission d'un message à l'attention du titulaire du compte, comportant un certains nombres d'informations collectées lors de vérifications effectuées dans les étapes 501 et 503 (adresse IP, authentification etc...) ainsi qu'à l'initialisation d'un compteur destiné à la mise en œuvre de la fonction de temporisation donnant une opportunité à ce dernier de bloquer l'accès à son compte.

Puis, dans une étape 505, le procédé procède à l'affichage, sur le système 20 du médecin urgentiste du Profil Médical d'Urgence (PMU) stocké sur le serveur distant (50). Comme précédemment, l'on pourra prévoir l'ouverture du fichier électronique de type PDF directement dans la langue d'accueil du service d'urgence.

Puis, dans une étape 506, le procédé procède à l'identification d'un premier tiers de confiance préalablement stocké dans la zone confidentielle protégée. L'identification du tiers de confiance étant stockée dans la zone protégée du serveur, celle-ci pourra donc être beaucoup plus riche et variée que celle consistant à inscrire un numéro de téléphone « anonyme » dans le Profil Médical d'Urgence (PMI), et qui serait potentiellement exposé à des abus d'utilisation.

Puis, dans une étape 507, le serveur sécurisé transmet automatiquement une requête au tiers de confiance désigné dans la liste stockée sur le dossier médical, suivant une forme quelconque. De préférence, la requête prendra la forme d'un courrier électronique transmis à l'adresse déclarée du tiers de confiance, éventuellement doublé d'un message d'un message de type SMS à l'attention du téléphone mobile du tiers de confiance.

De manière générale, tout message électronique pourra être envisagé.

Dans un mode de réalisation particulier, le tiers de confiance, ayant procédé à son enregistrement préalable auprès du serveur sécurisé (50) - et ce notamment grâce à sa propre carte médicale reçue pour le service - dispose d'une application au sein de son téléphone mobile ou de sa tablette tactile permettant l'affichage d'une interface d'urgence particulièrement explicite.

En particulier, l'interface permet au tiers de confiance de prendre connaissance de l'identité du service d'urgence requérant l'accès au dossier médical du patient considéré, éventuellement soutenues par des informations factuelles diverses (adresse IP, nom du médecin urgentiste, numéro de la carte Professionnelle de santé, géolocalisation du service d'urgence), ainsi que des coordonnées téléphoniques du médecin urgentiste sollicitant l'accès au dossier médical confidentiel.

Dans un mode de réalisation particulier, l'on prévoit, dans une étape 508, une possibilité d'échanges complémentaires entre le tiers de confiance et le système 20, de préférence via le serveur sécurisé 50. En particulier, l'on pourra prévoir, dans l'interface graphique de l'application installée dans le système 10 du tiers de confiance, la possibilité de transmettre via un bouton approprié et très clairement identifié, une photographie du titulaire du compte, qui pourra être, le cas échéant, transmise au médecin urgentiste via le serveur sécurisé 50 pour lui permettre de confirmer, le cas échéant, que son patient est bien le titulaire du dossier médical sollicité.

L'on pourra clairement envisager tout autre échange complémentaire entre le système 20 et le système 10, centralisé en tant que de besoin par le serveur 50. D'une manière générale, il pourra être utile que le serveur sécurisé serve d'intermédiaire à cet échange de manière à permettre la traçabilité des échanges et, par suite, une sécurité renforcée contre les fraudes avérées.

Au terme de cet échange, le tiers de confiance est en mesure de décider, au moyen notamment d'un jeu d'icônes/boutons très explicites affichés sur son téléphone mobile (par exemple), d'autoriser ou non l'ouverture du dossier médical confidentiel stocké sur le serveur 50, ce qui se traduit par l'envoi d'un message vers le serveur sécurisé 50 comportant la clé de substitution du tiers de confiance.

Dans une étape 509, le serveur 50 teste la présence de la clé de substitution reçue du tiers de confiance et, si celle ci est bien présente, le procédé poursuit avec l'étape 599 permettant l'ouverture du dossier médical confidentiel.

Au contraire, si la clé de substitution n'est pas reçue, le serveur 50 procède, dans une étape 510, à la fin de la procédure d'accès au dossier médical confidentiel, sans permettre son ouverture.

Comme précédemment, la clé de substitution pourra être implicite à l'envoi d'un message d'autorisation par le tiers de confiance, en sorte que ce message pourra être décodé par le serveur distant comme étant une manifestation de volonté explicite formalisant une clé de substitution valide pour accéder au dossier médical stocké sur le serveur sécurisé.

D'une manière générale, comme cela a déjà été évoqué, il est d'un grand intérêt que le serveur 50 conserve la trace de toutes les tentatives d'accès au dossier médical, ainsi que les échanges de messages et requêtes qui se sont produits, de manière à pouvoir constituer un dossier probatoire en cas de fraude avérée. En particulier, l'étape 510 peut s'achever par l'envoi d'un document récapitulatif complet au titulaire du dossier médical de manière à l'informer complètement des circonstances de temps et de lieux de la tentative d'accès à son dossier médical.

### 4. Quatrième mode de réalisation : le Profil Médical d'Urgence (PMU) stocké sur la carte médicale du patient

L'on décrit à présent un mode de réalisation particulier basé sur l'utilisation d'un support physique sophistiqué, sous la forme d'une carte médicale dotée d'une électronique propre permettant le stockage direct du Profil Médical d'Urgence (PMU).

Une telle carte, représentée par la référence 90 dans la figure 3, peut servir indifféremment pour le stockage du Profil Médical d'Urgence (PMU) de son propre titulaire et comporter également les programmes et données requis pour le stockage de la clé de substitution pouvant servir pour l'une des procédures d'accès sécurisées décrites dans la présente demande de brevet.

Accessoirement, et cela est un avantage important de ce quatrième mode de réalisation, la mémoire de stockage présente sur la carte médicale peut servir opportunément au stockage d'autres types de Profils Médicaux, tel que mentionnés précédemment, à savoir :
- un Profil Médical Handicap, destiné au personnes souffrant d'un handicap ;
- un Profil Médical Sport pour les sportifs ;
- un Profil Médical Chronique pour les sujets atteints de maladies chroniques ;
- un Profil Médical « baby », enfants, séniors.... pour les enfants etc...

Par ailleurs, les circuits électroniques présents sur la carte pourront fort opportunément servir à des procédures de synchronisation avec le serveur sécurisé 50. En effet, suite à des consultations et/ou examens effectués par le patient, les résultats de ces examens pourront être temporairement et avantageusement stocké sur la carte médicale, dans l'attente d'un téléchargement sur le site sécurisé 50.

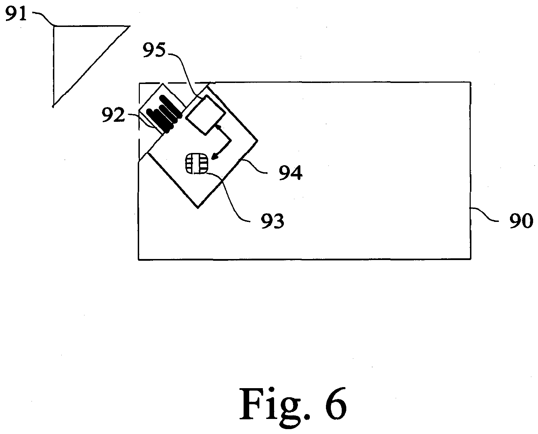

De préférence, comme cela est illustré dans la figure 6, la carte médicale se présente sous la forme d'une carte à puce 90 (dite *SMARTCARD*), ayant un facteur de forme format ID-1 de dimension 85,60 × 53,98 mm, et comportant une puce 93 incorporée suivant la normalisation ISO7816 intégrant des données d'identification sécurisées. Par ailleurs la carte médicale comporte un port USB 92 pour la communication série avec l'ordinateur 10 du patient, et des moyens de stockage 95 destinés au stockage d'un code d'exécutable pour la mise en oeuvre des fonctionnalités décrites ci-après, et notamment les accès sécurisés au serveur DM 50. D'une manière générale, les circuits électroniques actifs de la carte 90 pourront être disposés au sein d'une surface 94 intégrant les différents composants électroniques, dont la puce 93 noyés dans le matériau constitutif de la carte. Un capuchon 91 situé dans un angle de la carte 90 permet de recouvrir le port USB lorsque celui-ci n'est pas utilisé.

Dans un mode de réalisation préféré, la carte médicale présente un connecteur USB permettant une connexion directe, sans nécessiter de lecteur, à un port USB du système 10. Ce système présente l'avantage de correspondre au mode de connexion le plus répandu.

Dans le but d'éviter des altérations frauduleuses du contenu de la carte médicale, tous les fichiers stockés sur cette carte seront opportunément signés au moyen de la signature du serveur sécurisé (par exemple la clé privée de ce dernier), permettant d'assurer ainsi une intégrité des données stockées sur cette carte et éviter toute altération de son contenu.

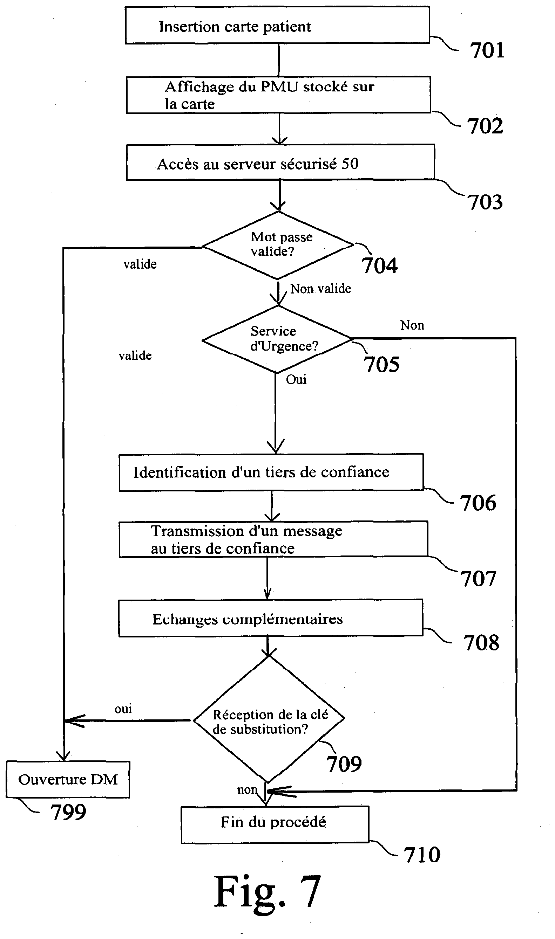

L'on décrit à présent, en relation avec la figure 7, un procédé d'accès au dossier médical suivant un quatrième mode de réalisation.

Dans une étape 701, le procédé démarre par l'insertion et la détection de la carte médicale 90 du patient dans le lecteur 21 du système 20 du médecin urgentiste.

Cette détection de la carte provoque la mise en œuvre et l'exécution d'un code informatique stocké sur la carte permettant l'ouverture immédiate du Profil Médical d'Urgence (PMU) du patient, dans une étape 702.

Puis, dans une étape 703, sur demande du médecin urgentiste, le procédé procède à un accès au serveur sécurisé distant 50. Il est à noter que dans ce quatrième mode de réalisation, la carte médicale comportera tous les informations utiles permettant un accès automatique au serveur depuis une interface spécifique, sans que le médecin urgentiste n'ait à saisir son lien U.R.L (*Uniform Ressource Locator*).

Puis, dans une étape 704, le serveur sécurisé vérifie la présence d'un mot de passe ou la validité d'un mot de passe saisi et, dans le cas d'un mot de passe valide, le serveur distant provoque, dans une étape 799, l'ouverture du dossier médical complet au moyen de l'affichage des informations nominatives mais également confidentielles.

Dans le cas contraire, le procédé poursuit avec une étape 705 au cours de laquelle le procédé vérifie que l'on se trouve bien dans une procédure d'urgence, notamment en testant l'authenticité du système 20 requérant l'accès au dossier médical. Comme précédemment, l'on pourra utiliser tout procédé d'authentification forte, notamment basé sur la Carte Professionnelle de Santé, sur l'identification du système requérant (enregistrement du médecin de santé), voire par la reconnaissance matérielle du système 20 sollicitant l'accès au dossier médical.

Si l'étape 705 n'aboutit pas, alors le procédé va directement à l'étape 710 correspondant à la fin de la procédure d'accès, au terme de laquelle, dans un mode de réalisation particulier, un courriel de synthèse est transmis au titulaire de la carte.

Si la vérification de l'étape 705 aboutit, alors le procédé poursuit avec une étape 706 au cours de laquelle le serveur sécurisé distant 50 procède à l'identification d'un premier tiers de confiance préalablement stocké dans la zone confidentielle protégée.

Puis, dans une étape 707 le serveur sécurisé transmet automatiquement une requête au tiers de confiance désigné, qui pourra à nouveau prendre la forme d'un courrier électronique transmis, éventuellement doublé d'un message d'un message de type SMS à l'attention du téléphone mobile du tiers de confiance.
Comme précédemment, l'on pourra envisager une application préalablement installée dans le système 10 du tiers de confiance, notamment dans son téléphone mobile intelligent.

Alternativement, et c'est un avantage de ce quatrième mode de réalisation, il suffira au tiers de confiance de procéder à l'insertion de sa propre carte médicale dans le lecteur de carte d'un système accessible pour trouver localement, stockées sur sa propre cartes, les ressources informatiques et les données utiles pour la prise en charge de la procédure d'autorisation et, le cas échéant, l'envoi de la clé de substitution au serveur sécurisé 50.

Comme auparavant, le code informatique stocké sur la carte médicale du tiers de confiance, ou l'application résidant dans son téléphone mobile intelligent pourra stocker et enregistrer les informations reçues du serveur 50 concernant notamment l'identité du service d'urgence requérant l'accès au dossier médical ainsi que toutes informations utiles ou circonstancielles de nature à permettre au tiers de confiance de se déterminer par rapport à la demande qu'il lui est présentée.

Comme précédemment, l'on pourra envisager, dans une étape 708 une possibilité d'échanges complémentaires entre les deux systèmes 10 et 20, notamment via le serveur sécurisé 50 , lesquels échanges pourront être enregistrés sur ce dernier - mais également sur les supports physique 90 de manière à en permettre la traçabilité.

Au terme de cet échange, le tiers de confiance est donc, comme auparavant, en mesure de décider s'il convient ou non d'accepter la demande d'accès au dossier médical confidentiel, et ce en transmettant la clé de substitution requise par le serveur sécurisé 50.

Dans une étape 709, le serveur 50 teste la présence de la clé de substitution reçue du tiers de confiance et, si celle ci est bien présente, le procédé poursuit avec l'étape 799 permettant l'ouverture du dossier médical confidentiel.

Au contraire, si la clé de substitution n'est pas reçue, le serveur 50 procède, dans une étape 710, à la fin de la procédure d'accès au dossier médical confidentiel, sans permettre son ouverture et en avisant le titulaire de l'accès avorté.

Comme précédemment, l'on pourra également considérer comme étant une clé de substitution valide toute manifestation de volonté formalisée par l'un des tiers de confiance, et notamment l'envoi d'un SMS (en réponse à un SMS transmis par le serveur sécurisé), d'un courriel (en réponse à un courriel transmis par le serveur sécurisé) ou un appel vocal, etc... D'une manière générale, cette clé de substitution aura pour objectif de valider/matérialiser l'accord du tiers de confiance auprès du serveur sécurisé qui déclenchera la procédure d'ouverture du Dossier Médical confidentiel, soit de manière automatisée, soit par l'envoi au serveur distant d'un mot de passe aléatoire et temporaire SMS, courriel...) Dans cette hypothèse, le test de l'étape 709 aboutira dès lors qu'un message SMS ou courriel etc... sera reçu par le serveur distant en réponse à la notification de l'étape 707.

### 5. Cinquième mode de réalisation : procédure d'obtention de la clé de substitution directement gérée par le système requérant

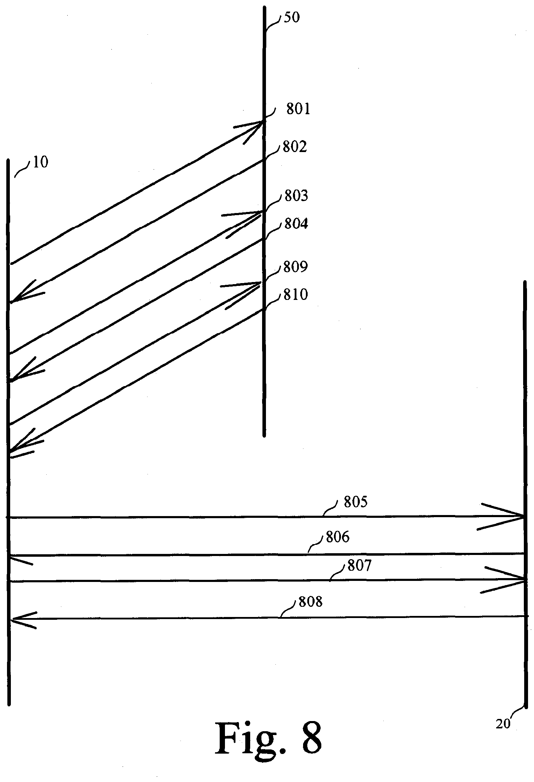

Afin d'illustrer les modes de réalisation possibles qui peuvent être envisagées, dans la grande diversité de leur combinaisons, l'on va décrire à présent, en relation avec la figure 8 un cinquième mode de réalisation dans lequel l'obtention de la clé de substitution est directement gérée par le système 20 du médecin urgentiste.

D'une manière générale, le procédé est conforme à la procédure décrite en relation avec la figure 7, à l'exception toutefois que les étapes 705-707 sont directement effectuées entre les deux systèmes 20 et 10.

Ce qui a pour effet de réduire la quantité de message échangés avec le serveur sécurisé, comme ce que l'on voit dans la figure 8 montrant :
Messages 801-802 : correspond à la requête d'accès au serveur sécurisé et sa réponse de ce dernier:
Messages 803-804 : correspond à la procédure de vérification du mot de passe (par hypothèse sans succès)
Message 805 : requête transmise par le système 20 au système 10 pour solliciter la clé de substitution
Messages 806-807 : illustrent un échange complémentaire entre les systèmes 10 et 20 ;
Message 808 : correspond à l'envoi par le système 10 de la clé de substitution ;
Message 809 : le système 20 peut transmettre la clé de substitution au serveur sécurisé et, en réponse (message 810), obtient un accès au dossier médical du titulaire.

Comme on le voit, l'invention permet de multiples réalisations permettant la combinaison du Profil Médical d'Urgence et l'intervention du tiers de confiance.

L'on notera que les procédures décrites précédemment pourront se dérouler successivement (ou en parallèle pour un gain de temps) pour toute la liste de tiers de confiance stockées dans le dossier médical et ce, jusqu'à épuisement de la liste. Un homme du métier pourra donc aisément adapter les procédures décrites à la mise en oeuvre de boucles de traitement permettant d'épuiser la liste des tiers de confiance.

Dans un mode de réalisation particulier, en cas d'épuisement de la liste des tiers de confiance, l'on prévoit la possibilité de substituer, au tiers de confiance, un organisme de confiance qui pourra, en ultime ressort et dans les cas particulièrement graves dans lesquels le pronostic vital du titulaire est engagé, provoquer l'ouverture du dossier médical de ce denier.

### 6. sixième mode de réalisation : Gestion locale du PMP sur un support externe local (carte/clé USB ou autre support) grâce à un applicatif embarqué.

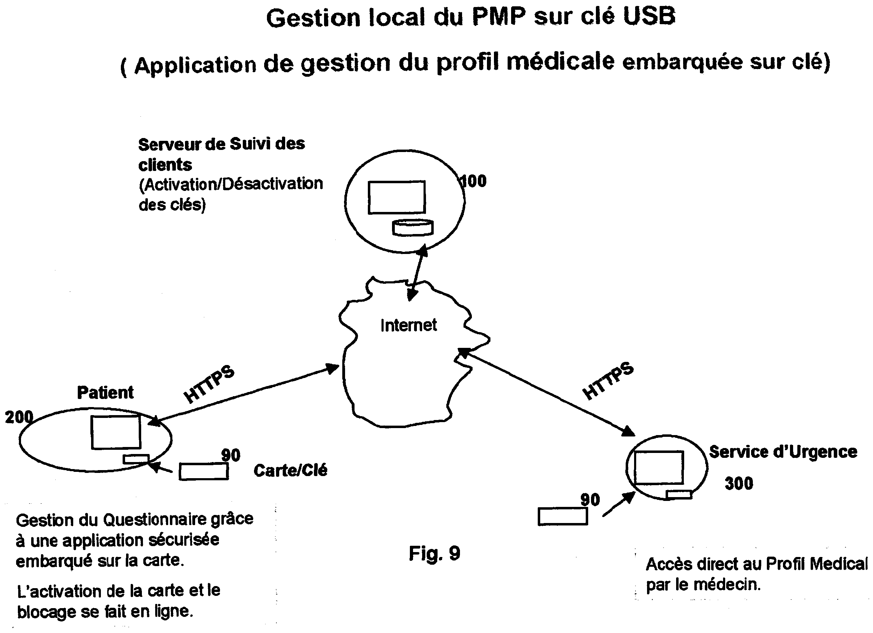

Afin d'illustrer d'autres modes de réalisation possibles qui peuvent s'envisager de manière simplifiée et locale à l'aide d'un applicatif embarqué sur carte/USB, on va décrire à présent, en relation avec la figure 9, un sixième mode de réalisation dans lequel l'affichage du questionnaire médical se fait grâce à un logiciel embarqué directement sur le support externe.

La figure 9 illustre plus particulièrement la procédure qui est mise en oeuvre, pour constituer localement le profil médical personnel grâce à un applicatif intégré protégé sur la carte (90) et, par la suite, générer automatiquement le Profil Médical personnel .Dans ce mode de réalisation aucune donnée médicale n'est stockée sur le serveur. Seul une clé d'activation de la carte est délivrée par le serveur de suivi de patients (100).

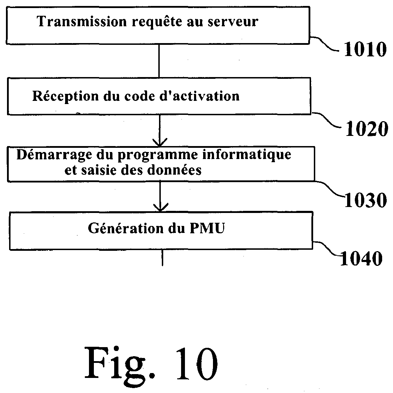

Le procédé est plus particulièrement décrit dans la figure 10. Dans une première étape 1010, le patient (200) fait une demande d'activation de sa carte (90) en ligne (en insérant sa carte la 1 ère fois) auprès du serveur (100) . A cet effet, le procédé procède à la transmission d'une requête destinée à un serveur externe, lequel serveur lui permet de remplir sa fiche personnel (Nom, Prénom,..), ensuite délivre une clé ou un code d'activation de la carte qui lui est transmis par des moyens sécurisés (SMS, Courrier,..), lequel est réceptionné dans une étape 1020. Le patient (200) en insérant sa carte permet le lancement automatique, dans une étape 1030, d'un applicatif embarqué permettant la saisie et le stockage des données . Grâce à ce dernier, et en saisissant le login mot de passe, le patient procède à la saisie d'un Questionnaire Médical conçus de manière à permettre la collecte de ses données médicales personnelles la saisie étant associée à l'attribution d'un caractère confidentiel ou non à chacune des informations saisies par le titulaire du dossier médical. Ces informations sont stockées automatiquement dans une base de données locale sur la dite carte (90). Un profil médical personnel est ensuite généré automatiquement sur la carte (sous format PDF et en plusieurs langues).

Ainsi, dans une situation d'urgence par exemple, un médecin urgentiste (300) qui aura à prendre en charge un patient inanimé ou en état de choc, aura la possibilité d'accéder directement à son Profil Médical d'Urgence (PMU), simplement en se saisissant de la carte du patient, et en ouvrant le document profil médical.Un premier niveau d'affichage sans mot de passe permet au médecin d'avoir les informations publiées non confidentielles par le patient au sein d'un dossier médical d'urgence généré dans l'étape 1040 du procédé de la figure 10.Un deuxième niveau d'affichage permet au médecin de voir la totalité des informations en saisissant le mot de passe du patient, ou alternativement via la procédure du tiers de confiance décrite précédemment.

### 7. Développements supplémentaires et considérations finales

L'on expose ci-après des développements spécifiques concernant :
7. 1) les niveaux d'applicatifs utilisables dans la carte médicale
7.2) la sécurisation de la carte médicale et la sauvegarde des données
7.3.) les avantages de la solution préconisée

### 7.1. Niveaux d'applicatifs envisageables :

Différents niveaux d'applicatifs pourraient être proposés :

### 7.1.1. Applicatif de niveau 1

Questionnaire:
- Pour l'utilisateur, un double questionnaire :
   ∘ un questionnaire à données publiables
   ∘ un questionnaire à données protégées
- Les questionnaires pourraient être remplis successivement ou séparément par un login/passeword délivré après initialisation /serveur
- Le questionnaire publiable s'afficherait automatiquement sous forme de fichier électronique de type PDF Le questionnaire protégé s'afficherait sous son aspect initial avec le login/passeword (on pourrait passer de manière dynamique et simple de l'un à l'autre.)

Pièces jointes
- Le questionnaire publiable permettrait d'afficher un nombre de pièces jointes limitées au minimum (la photo d'identité, le groupe sanguin, les vaccinations et les allergies si le patient possède un document médical en cas d'allergie grave)
- Le questionnaire protégé aurait droit à toutes les pièces jointes correspondantes aux items demandés.

Cette carte répondrait ainsi, déjà, à un niveau de confidentialité élémentaire, mais simple.

Dynamisme des écrans, personnalisation, sécurité et validation médicale.
- Il n'y aurait pas de présentation dynamique dédiée à l'urgentiste, mais un affichage PDF
- Sur le plan de la personnalisation, elle serait limitée au choix de ne pas répondre à une question, et serait guidée par chacun des 2 questionnaires sur la publication des données. Une zone de texte permettrait de compléter les informations dans chacun des 2 questionnaires, offrant ainsi un espace de liberté de publication ou de confidentialité illimitée
- Sur le plan de la sécurité : protection de la carte (intrusion de virus, destruction/piratage du logiciel...), mais aussi délivrance du mot de passe perdu et si possible création d'un nouveau mot de passe obligatoire si perte de carte

Validation médicale :
- Les pièces jointes, même en niveau 1 sur le questionnaire publiable, déterminent déjà un niveau de validation médicale.
- Les pièces jointes sur le questionnaire protégé peuvent faire l'objet d'une adaptation automatisée à l'espace de stockage.
- Concernant cette validation médicale, je propose d'afficher une zone de «collaboration médicale »

Cette zone indiquerait alors :
- S'il y a des pièces jointes ou non, le nombre de pièces jointes, et éventuellement donner accès directement au fichier des pièces jointes, suivant le questionnaire dans lequel on se trouve.
- Elle indiquerait si c'est le cas, que ce dossier/questionnaire a été rempli en collaboration ou avec l'aide d'un médecin. Si c'est le cas, il serait indiqué le nom du médecin, et ses coordonnées. Si ce n'est pas le cas, la zone serait vide. Une historisation des validation est à prévoir.

### 7.1.2. Applicatif de niveau 2 :

- Support externe à forte capacité de stockage
- Questionnaire unique, mais dynamique sur plusieurs chapitres
- Questionnaire permettant directement le marquage des informations publiables et importantes. Personnalisation de la synthèse.
- Affichage dynamique pour l'urgentiste des données qui ne sont plus sous forme de PDF, mais dans un masque dynamique où chaque chapitre contient en face les éléments importants et publiables (imprimable), et sur lequel l'urgentiste peut en cliquant directement sur le chapitre accéder par un loguin /mot de passe aux informations protégées. Cela lui permettra d'aller directement sur les chapitres qui l'intéressent en cas d'urgence/manque de temps.
- Sur cette carte le niveau de sécurité peut être renforcée, avec un système plus élaboré de blocage de carte et ou restitution du mot de passe oublié.
- Concernant les pièces jointes, celles-ci seraient accessibles par lien direct, sans passer par un fichier de classement.
- Historisation obligatoire d'une validation médicale.
Ces différents modes de réalisation permettent de développer des niveaux successifs avec des objectifs différents en termes de coût d'hébergement, de coût du support externe (capacité), mais aussi de législation vis-à-vis des données médicales (simple déclaration ou demande d'autorisation CNIL...).

L'esprit de l'invention reste toutefois le même, en donnant à chaque niveau
- Un questionnaire médical adapté et simple d'utilisation par le patient pour lui permettre de déterminer son Profil Médical Personnel.
- Une validation médicale par pièces jointes ou certificat de co-validation par un médecin authentifié.
- Une présentation dynamique, facile et efficace pour les urgentistes
- L'affichage de données publiables personnalisées par le patient lui-même
- Un mot de passe qui protégerait l'accès aux données confidentielles.
- Une sécurisation en cas de perte ou de vol du support.

L'intérêt de ces différents modes de réalisation est de proposer des solutions techniques qui peuvent s'adresser à des intérêts médicaux distincts :
- Niveau 1 : Un niveau financièrement attractif à l'achat et sans récurrence, « CNIL déclaratif », mais médicalement validé et protégé par un Login/passeword pour les données confidentielles.
   Ce niveau comprendrait une sécurisation concernant la perte ou le vol. La délégation à un tiers de confiance en cas de nécessité serait possible. Ce produit correspondrait typiquement au besoin du profil médical d'urgence
- Niveau 2 : Un produit hébergé et sécurisé, permettant la récurrence et les statistiques, avec validation médicale forte (authentification) plus adapté à un profil médical plus large (sénior, handicap, maladies chroniques..) et un « échange » médecin patient plus élaboré, et avec des accès différenciés.
- Niveau 3 : Un produit qui mixte les 2 premiers niveaux, irréprochable d'un point de vue médical et sécurité, permettant les échanges transfrontaliers et l'affichage hors connexion d'un véritable Profil Médical Personnel. Ce produit correspondrait plus à un suivi du dossier par le médecin à travers le patient.

Cette innovation présente ainsi une solution :
**Simple,** car la solution décrite est simple à utiliser, tant pour le patient que pour le médecin.
**Essentielle,** car elle délivre des informations essentielles, voire vitales, et ce, à des cibles précises (personnes à risques = urgences, handicap, sénior, sportifs, femmes enceinte...), et non à toute la population à la différence du Dossier Médical Personnel.
**Personnelle,** au sens de personnalisable par le patient lui-même, qui prend alors toute la maîtrise de la présentation de ses propres données, celles qu'il souhaite publier ou celles qu'il préfère protégées, ou même ne pas divulguer.
**Sécurisée,** tant au niveau des protections techniques que de la notion de confidentialité qui doit être respectée.
**Validée,** au sens d'une validation médicale, mais aussi d'une validation CNIL

### 7.2.. Sécurisation de la carte et sauvegarde des données

### 7.2.1. Sauvegarde des données.

Étant donné que les données saisies par le patient sont stockées sur la carte, il pourra être opportun d'envisager une procédure de sauvegarde, par exemple suivant l'une des trois modalités ci-dessous :
- l'utilisation d'une connexion ponctuelle au serveur central, à la demande du patient pour permettre le stockage des données, et notamment dans un fichier anonyme et crypté. Ce fichier de sauvegarde pourra être référencé par un code spécifique au patient lié à sa propre carte. Ainsi, le patient peut ainsi à tout moment se connecter au serveur central et restituer ses propres données en cas de perte de la carte et la délivrance d'une autre.
- la mise en œuvre d'une sauvegarde locale, à la demande du patient pour que à tout moment il puisse sauvegarder ses données sur un autre support (PC, tablette etc...) Ces données seront stockées dans un fichier anonyme et cryptées sur le support choisi, par exemple référencé par un code spécifique au patient et non lié à sa propre carte. De cette manière, le patient pourrai restituer à tout moment ses propres données en cas de perte de la carte et la délivrance d'une autre.
- la mise en œuvre d'une procédure de sauvegarde locale automatique en utilisant un système de miroir fonctionnant avec l'ordinateur personnel du patient. Ce système permettra à celui-ci de générer une copie de ses données et même de l'applicatif, lesquelles seront stockées sur son ordinateur personnel.

Ainsi, grâce à la mise en œuvre de l'une ou l'autre de ces modalités, le patient pourra à tout moment restituer ses propres données en cas de perte de la carte et la délivrance d'une autre. Il pourra aussi utiliser son propre ordinateur PC comme support de saisie et permettre un envoi de données anonymes sur le serveur pour servir divers calculs statistiques.

Cette solution permettra également un possible dialogue avec d'autres logiciels installés sur l'ordinateur du patient qui seraient susceptibles d'enrichir le Profil Médical par des éléments de suivi médical (radiologie, biologie, suivi tensionnel, suivi de le fréquence cardiaque pour la pratique du sport, suivi du diabète...)

### 7.2.2- Sécurisation de la carte

Le but est de sécuriser le logiciel hébergé sur la carte

### 7.2.2.1. Partitionnement de la clé USB :

L'on peut considérer les 3 partitions suivantes:

### 7.2.2.1.1. - partition de boot

- Format CD-Rom, lecture seule, non modifiable, lecture automatique par le système
- contenant le programme de boot.

### 7.2.2.1.2 - partition du programme

- Format FAT32 ou NTFS
- cryptée et cachée,
- en lecture-écriture (pour les mises à jour).
- contenant le logiciel et le questionnaire.

### 7.2.2.1.3. partition des données

- Format FAT32.
- Cryptée et cachée.
- prenant toute la place restante disponible de la clé USB.
- Saturée de volume virtuel (eux-mêmes crypté), de 4Go maximum (jusqu'à remplissage de la partition).
- le 1er volume virtuel contiendra la base de données
- les autres contiendront les éventuels fichiers attachés au questionnaire par l'utilisateur (jusqu'à saturation de tous les volumes virtuels).

### 7.2.2.2) Ouverture des partitions :

7.2.2.2.1. Le système d'exploitation ouvrira automatiquement (sauf s'il est désactivé dans les préférences) la partition de boot, et exécutera le logiciel de boot (créé par nos soins).

7.2.2.2.2. Ce logiciel de boot décryptera la partition du programme (mais la conservera masquée) et exécutera le programme principal.

7.2.2.2. 3 Celui-ci, dans le cas où l'utilisateur a saisi ses identifiant et mot de passe, décryptera la partition des données (mais la conservera masquée), ainsi que les volumes virtuels y figurant.

### 7.2.2. 3) Protection contre les virus :

- La seule partition accessible à tout moment est la partition de boot, en lecture seule non modifiable (donc protégé).
- Les deux autres partitions sont cryptées et masquées.
- Les deux autres partitions, ainsi que les volumes virtuels, contiendront (une fois décryptés et accessibles) des fichiers arrêtant les virus (dossier «autorun.inf» en lecture seul ; dossiers «recycled» et «recycler» masqués ; ...)
- La saturation des partitions par des volumes virtuels empêchera un virus d'avoir de la place pour s'installer sur la clé USB.

### 7.2.2. 4) Protection contre les erreurs de l'utilisateur :

- Aucun accès « standard » à l'application ni aux données ; évitant une suppression involontaire ou une modification manuelle des fichiers.
- Accès seulement à la partition de boot, en lecture seule et ne contenant que le logiciel de boot.
- La totalité des données, ainsi que tous les fichiers attachés par l'utilisateur au questionnaire, ne sont accessibles que par l'application principale.
- Les profils, public et confidentiel, ne sont accessibles que par l'application.

### 7.2.2. 5) Protection contre l'accès aux données confidentielles :

- Les données sont stockées dans une base de données cryptée ; elle-même dans une partition cryptée et masquée.
- La génération du profil confidentiel se fait toujours « à la volée », uniquement si l'utilisateur à bien saisi sont identifiant et mot de passe, et n'est jamais stocké.

### 7.2.2. 6) Protection contre les vols ou perte :

- Une personne mal intentionnée ne pourra accéder aux données confidentielles que s'il connaît l'identifiant et le mot de passe du propriétaire de la carte.
- Cette même personne n'accédera qu'aux données publiques de la carte, tout comme par défaut le personnel médical.
- Si cette personne demande une réinitialisation du mot de passe, le code de réinitialisation correspondant (unique par carte) sera envoyé au propriétaire de la carte (adresse email stockée et conservée au moment de l'activation de la carte).

### 7.3. Considérations finales : des avantages significatifs

Les solutions préconisées, et notamment celle de carte médicale décrite précédemment, offrent des avantages particulièrement décisifs, notamment parce qu'il s'agit d'une solution assurant la centralisation et le partage de données médicales numérisées dans un contexte et un milieu comportant, par essence des freins et résistances importantes :

### 7.3.1. Les résistances liées au milieu médical

- Les inventeurs ont constaté que, dans bien des cas, les professionnels de la santé peuvent avoir du mal à adhérer à un projet de l'enregistrement centralisé des informations médicales pour diverses raisons : manque de temps, double saisie logicielle, problématique liée à la rémunération d'une activité administrative et non médicale, crainte de «partager» l'information...).

D'une manière générale, les médecins apparaissent, dans l'ensemble, peu motivés pour rendre ce service d'intérêt publique qui n'a pas encore reçu de module économique clair, au moins pour eux.

Dans la perspective de la mise en place d'un grand projet de la numérisation des dossiers médicaux, il est apparu aux inventeurs que l'initiative pourrait venir plus aisément des patients.

### 7.3.2. les résistances économiques

D'un point de vue économique, la centralisation apparaît coûteuse en terme d'hébergement (Agrément nécessaire pour les hébergeurs de données médicales). L'interfaçage des logiciels est long et onéreux entraînant des mises à jour incessantes pour s'adapter à un interfaçage en constante évolution.

### 7.3.3. Les résistances techniques

D'un point de vue technique, la difficulté de gestion des droits d'accès est extrêmement complexe à mettre en oeuvre pour un échange entre thérapeute de toute spécialités et de toute catégories (médicaux, paramédicaux...).

La sécurisation de cette centralisation nécessite des moyens énormes pour une «inviolabilité » encore incertaine.

On ignore encore quelle solution technologique (en constante évolution) va remporter le maximum d'adhésion chez les médecins :
∘ ordinateur classique qui permet aujourd'hui la lecture de l'imagerie via un DVD / DICOM,
∘ les tablettes tactiles ou smartphones sans connective possible,
∘ des applications locales synchronisables
∘ ou des logiciels connectés purement web risqués en l'absence de réseau internet...

Les paris technologiques et commerciaux apparaissent ainsi bien risqués.

### 7.3.4. Les résistances d'un point de vue juridique

- D'un point de vue juridique, enfin, la complexité des réglementations concernant l'hébergement des données de santé, dès l'instant où celles-ci sont recueillies par le praticien, est un frein certain et important : consentement patient, authentification forte, échanges transfrontaliers entre pays de niveaux de protection différent, exhaustivité des données enregistrées, confidentialité des accès...
- Cette complexité est accrue en France par la notion d'hébergement chez un hébergeur agréé qui certes, rajoute une protection supplémentaire à la sécurisation des données, mais élève considérablement les coûts d'hébergement.

Au regard de tous ces freins et résistances, les inventeurs ont découvert que les solutions préconisées et décrites précédemment, notamment avec la carte médicale personnelle, sont susceptible d'apporter une solution plus rapide à être mise en œuvre pour les situations d'urgence et des cibles prioritaires en attendant la maturation du projet d'hébergement centralisé destiné à l'ensemble de la population, maturation qui devrait prendre encore plusieurs années pour une complète efficacité.

C'est pourquoi la solution de la carte médicale qui a été décrite précédemment, en combinaison avec les procédés décrits et revendiqués, apportent une solution particulièrement avantageuse puisqu'elle :
- implique significativement le Patient (qui affecte ou non un caractère confidentiel aux informations saisies dans le formulaire, et comportant une validation médicale)
- peut éviter dans une certaine mesure un hébergement centralisé et systématique de toutes les données médicales, puisque certaines d'entre elles pourront être stockées sur la carte ;
- Concerne l'essentiel du dossier, mais pas son intégralité (Profil plutôt que Dossier)
- S'adresse en priorité à des cibles sensibles ou prioritaires
- Est matérialisable et facilement accessible
- Peu onéreuse pour les patients (et sans coût pour l'état)
- Et, surtout, qui respecte toute la législation sur la sécurisation et la confidentialité vis-à-vis du patient.

Tels sont des avantages significatifs qui devraient contribuer à une généralisation rapide des solutions décrites précédemment.

## Revendications

1. Procédé de génération d'un dossier médical numérique stocké sur un serveur sécurisé (50) et accessible depuis un premier système (10) via un réseau de communication, ledit dossier médical numérique comportant des données nominatives et confidentielles, ledit procédé comportant la génération automatique d'un dossier médical d'urgence, PMU, dénué de toute information nominative et confidentielle et destiné à être stocké sur un support physique détenu par le titulaire, le procédé comportant les étapes suivantes :
- création (101) d'un compte et génération d'un identifiant et d'un mot de passe ;
- création (102) d'un questionnaire administratif comportant des informations administrative nominatives ;
- création (103) d'un questionnaire médical destiné à collecter des données d'ordre médical, éventuellement accompagné d'annexes ou de pièces jointes validant ces données ;
- génération (105) d'une synthèse des données saisies via le questionnaire médical, en relation avec un outil de classement destiné à autoriser ou non l'introduction de chaque données saisie dans un Profil Médical d'Urgence librement accessible depuis ledit support,
**caractérisé en ce que** le procédé comporte les étapes suivantes :
- désignation (106) d'un ou plusieurs tiers de confiance ;
- génération automatique dudit Profil Médical d'Urgence, PMU, ne comportant que des données non nominatives et classifiées non confidentielles, ledit Profil Médical d'Urgence étant stocké sur une zone non protégée et accessible depuis ledit support physique les autres données nominatives et/ou confidentielles étant stockées dans une zone protégées dudit serveur sécurisé accessible au moyen de l'identifiant et du mot de passe ;
ledit Profil Médical d'Urgence comportant en outre un lien permettant d'accéder à un tiers de confiance en vue de l'obtention d'une clé de substitution utilisable en l'absence d'un mot de passe ou de tout autre moyen technique permettant la lecture du dossier médical confidentiel et/ou nominatif.

2. Procédé selon la revendication 1, **caractérisé en ce que** la clé de substitution résulte d'une expression de volonté dudit tiers de confiance de permettre l'accès au dossier médical, prenant la forme d'un message électronique, tel que SMS ou courriel, ou d'un appel vocal, ou d'un message électronique transmis par une application depuis le système dudit tiers vocal.

3. Procédé d'accès à un dossier médical numérique stocké sur un serveur sécurisé (50) et accessible depuis un premier système (10) via un réseau de communication, ledit dossier médical numérique comportant des données nominatives et confidentielles et généré par le procédé de la revendication 1, ledit procédé comportant les étapes suivantes :
- accès (200) audit serveur sécurisé (50) au moyen d'un support physique comportant un identifiant à l'exclusion de tout mot de passe ;
- présentation d'un identifiant et ouverture (201) d'une session avec ledit serveur sécurisé (50) ;
- vérification du mot de passe (202) et , en cas de mot de passe valide, ouverture du dossier médical ;
- en cas de défaut de mot de passe valide, transmission (203) optionnelle d'un courriel au titulaire du compte (203);
- affichage (204) du Profil Médical d'Urgence, PMU,
- identification d'un tiers de confiance (205);
- accès au tiers de confiance et obtention d'une clé de substitution (206);
- transmission de la clé de substitution (207) audit serveur sécurisé ;
- vérification (208) de ladite clé de substitution et, en cas de concordance avec les clés inscrites dans le dossier médical, ouverture du dossier médical.

4. Procédé selon la revendication 3 **caractérisé en ce que** l'accès au dossier médical numérique découle de la réception par le serveur sécurisé d'un message électronique, tel un SMS ou un courriel, transmis depuis le propre système attribué audit tiers de confiance.

5. Procédé selon la revendication 3, **caractérisé en ce que** l'ouverture du dossier médical numérique résulte de la réception par ledit serveur sécurisé d'un appel vocal reçu de la part dudit tiers de confiance.

6. Procédé selon la revendication 3, **caractérisé en ce que** l'accès audit serveur sécurisé via ladite carte mémoire ou carte USB provoque, en cas d'échec de ladite procédure de vérification de mot de passe, entraîne l'envoi d'un message électronique vers le ou l'un des tiers de confiance préalablement désigné par ledit titulaire de la carte mémoire.

7. Procédé selon la revendication 3 **caractérisé en ce qu'**il comporte les étapes :
- insertion et lecture (701) d'une carte non nominative comportant un Profil Médical d'Urgence, PMU, ne comportant aucune information médicales et aucune information confidentielle, ainsi qu'un lien permettant un accès audit serveur ainsi qu'à un ou plusieurs tiers de confiance ;
- Affichage (702) du Profil Médical d'Urgence sur un dispositif de traitement de données dudit dossier médical d'urgence ;
- accès (703) audit serveur sécurisé (50) ;
- vérification du mot de passe (704) et ouverture du dossier médical en cas de concordance du mot de passe présenté ;
- en cas de défaut de mot de passe, vérification (705) de l'authenticité du système réquérant l'accès, comme appartenant à un service d'urgence ;
- identification (706) d'un tiers de confiance ;
- transmission d'un message au tiers de confiance (707) sollicitant une clé de substitution ;
- obtention (708) de ladite clé de substitution et récupération d'une information d'identification dudit tiers de confiance ;
- utilisation (708, 709) de ladite information pour accéder à la zone protégée audit serveur distant.

8. Procédé de génération d'un dossier médical numérique destiné à être stocké sur un support amovible, tel qu'une carte à mémoire, comportant un espace de stockage ainsi qu'un programme informatique résidant sur ledit support,
ledit procédé comportant les étapes:
- transmission (1010) d'une requête destinée à un serveur externe dans le but d'obtenir une clé ou un code d'activation dudit programme informatique, ladite requête permettant un enregistrement administratif auprès dudit serveur;
- réception (1020) de la clé ou dudit code d'activation généré par ledit serveur ;
- démarrage (1030) dudit programme informatique permettant la saisie par le patient d'un dossier Questionnaire médical conçu de manière à permettre la collecte de ses données médicales personnelles, et le stockage de celles-ci dans une base de données disposée au sein dudit support de stockage, **caractérisé en ce que** :
- chaque donnée saisie est associée à un caractère également saisi par le patient pour autoriser ou non l'introduction de cette dernière dans un Profil Médical d'Urgence librement accessible depuis ledit support, et
- ledit profil médical personnel d'urgence correspondant au patient est généré, par exemple dans un format électronique pouvant être disponible en plusieurs langues, ce profil médical personnel d'urgence ne comportant que les seules données autorisées par le patient.

9. Procédé selon la revendication 8 **caractérisé en ce que** l'accès aux informations confidentielles du dossier médical peuvent être lues via un accès indirect au dossier médical numérique confidentiel et/ou nominatif via un tiers de confiance.

10. Procédé selon la revendication 8 ou 9, **caractérisé en ce que** ledit support comporte un lien vers ledit serveur sécurisé permettant un accès aux données nominatives et confidentielles stockées sur ledit serveur via une procédure de vérification de mot de passe.

11. Procédé selon la revendication 8, **caractérisé en ce qu'**il comporte les étapes :
- création (101) d'un compte et génération d'un identifiant et d'un mot de passe ;
- création (102) d'un questionnaire administratif comportant des informations administrative nominatives ;
- création (103) d'un questionnaire médical destiné à collecter des données d'ordre médical, éventuellement accompagné d'annexes ou de pièces jointes validant ces données ;
- génération (105) d'une synthèse des données saisies via le questionnaire médical, en relation avec un outil de classement destiné à assigner à chacune des données un caractère confidentiel ou non ;
- désignation (106) d'un ou plusieurs tiers de confiance ;
- génération automatique d'un Profil Médical d'Urgence, PMU, ne comportant que des données non nominatives et classifiées non confidentielles, ledit Profil Médical d'Urgence étant stocké sur une zone non protégée et accessible depuis un support externe comportant ledit identifiant, les autres données nominatives et/ou confidentielles étant stockées dans une zone protégées dudit serveur sécurisé accessible au moyen de l'identifiant et du mot de passe ;
ledit Profil Médical d'Urgence comportant en outre un lien permettant d'accéder à un tiers de confiance en vue de l'obtention d'une clé de substitution utilisable en l'absence d'un mot de passe ou de tout autre moyen technique permettant la lecture du dossier médical confidentiel et/ou nominatif

12. Procédé selon la revendication 11, **caractérisé en ce que** la clé de substitution résulte d'une expression de volonté dudit tiers de confiance de permettre l'accès au dossier médical, prenant la forme d'un message électronique, tel que SMS ou courriel, ou d'un appel vocal, ou d'un message électronique transmis par une application depuis le système dudit tiers vocal.

13. Support de stockage électronique pour un dossier médical numérique comportant un espace de stockage pour ledit dossier médical numérique, ainsi qu'un programme informatique résidant sur ledit support, adapté pour la mise en oeuvre des procédés définis dans l'une des revendications 8 à 12, ledit support comportant :
- des moyens de transmission d'une requête destinée à un serveur externe dans le but d'obtenir une clé ou un code d'activation dudit programme informatique, ladite requête permettant un enregistrement administratif auprès dudit serveur;
- des moyens de réception de la clé ou dudit code d'activation généré par ledit serveur ;
- des moyens pour démarrer ledit programme informatique permettant la saisie par le patient d'un dossier Questionnaire médical conçu de manière à permettre la collecte de ses données médicales personnelles, et le stockage de celles-ci dans une base de données disposée au sein dudit support de stockage, la saisie étant associée à l'attribution d'un caractère confidentiel ou non à chacune des informations saisies par le titulaire du dossier médical;
- des moyens pour générer automatiquement un profil médical personnel d'urgence correspondant au patient, par exemple dans un format électronique pouvant être disponible en plusieurs langues, ne comportant que les seules données auxquelles ont été attribuées un caractère non confidentiel.

14. Support selon la revendication 13 **caractérisé en ce qu'**il comporte des moyens permettant un accès indirect au dossier médical numérique confidentiel et/ou nominatif via un tiers de confiance.

15. Support de stockage se présentant sous la forme d'une carte comportant des circuits électroniques et des moyens de stockage permettant la mise en œuvre des procédures décrites dans les revendications 1 à 12, ainsi qu'un logiciel local en lien avec un serveur permettant de proposer les revendications dans un mode local avec applicatif embarqué.

## Patentansprüche

1. Verfahren zur Erzeugung einer digitalen Krankenakte, die auf einem gesicherten Server (50) gespeichert und von einem ersten System (10) aus über ein Kommunikationsnetz zugänglich ist, wobei besagte digitale Krankenakte personenbezogene und vertrauliche Daten umfasst, wobei besagtes Verfahren die automatische Erzeugung einer Krankenakte für dringliche Behandlung, PMU, umfasst, die keine personenbezogenen und vertraulichen Informationen enthält und dazu bestimmt ist, auf einem materiellen Träger des Inhabers gespeichert zu werden, wobei das Verfahren die folgenden Schritte umfasst:
- Schaffung (101) eines Kontos und Erzeugung eines Benutzernamens und eines Passworts;
- Schaffung (102) eines Verwaltungsfragebogens, der personenbezogene Verwaltungsinformationen umfasst;
- Schaffung (103) eines medizinischen Fragebogens, um medizinische Daten zu sammeln, eventuell mit Anhängen oder Anlagen, die diese Daten validieren;
- Erzeugung (105) einer Synthese der über den medizinischen Fragebogen erfassten Daten, in Zusammenhang mit einem Klassifizierungswerkzeug, das dazu bestimmt ist, die Einführung aller erfassten Daten in ein medizinisches Profil für Notfälle, das von besagtem Träger aus frei zugänglich ist, zu gestatten oder nicht,
**gekennzeichnet dadurch, dass** das Verfahren die folgenden Schritte umfasst:
- Angabe (106) eines oder mehrerer vertrauenswürdigen Dritten;
- automatische Erzeugung des genannten medizinischen Profils für Notfälle, PMU, das nur nicht personenbezogene Daten und als nicht vertraulich klassifizierte umfasst, wobei besagtes medizinische Profil für Notfälle in einem nicht geschützten Bereich gespeichert ist, der von besagtem physikalischen Träger aus zugänglich ist, die anderen personenbezogenen und/oder vertraulichen Daten sind in einem geschützten Bereich des genannten gesicherte Servers gelagert, der mittels des Benutzernamens und des Passworts zugänglich ist;
- besagtes medizinische Profil für Notfälle umfasst außerdem einen Link zum Zugang zu einem vertrauenswürdigen Dritten, zum Erhalt eines Ersatzschlüssels, der bei Fehlen eines Passworts oder jeglichen anderen technischen Mittels zur Ablesung der vertraulichen und/oder personenbezogenen Krankenakte verwendbar ist.

2. Verfahren nach Anspruch 1, **dadurch gekennzeichnet, dass** der Ersatzschlüssel ein Ausdruck des Willens des genannten vertrauenswürdigen Dritten ist, den Zugang zu der Krankenakte zu gestatten, der die Form einer elektronischen Nachricht annimmt, wie SMS oder E-Mail, oder eines Sprachanrufs, oder einer elektronischen Nachricht, die durch eine Anwendung von dem System des genannten Dritten aus übertragen wird.

3. Verfahren zum Zugang zu einer digitalen Krankenakte, die auf einem gesicherten Server (50) gespeichert und von einem ersten System (10) aus über ein Kommunikationsnetz zugänglich ist, wobei besagte digitale Krankenakte personenbezogene und vertrauliche Daten umfasst und durch das Verfahren von Anspruch 1 erzeugt wird, wobei das Verfahren die folgenden Schritte umfasst:
- Zugang (200) zu dem genannten gesicherten Server (50) mittels eines materiellen Trägers, der einen Benutzernamen umfasst, ausschließlich jedes Passworts;
- Vorstellung eines Benutzernamens und Eröffnung (201) einer Sitzung mit besagtem gesicherten Server (50) ;
- Prüfung des Passworts (202) und, bei gültigem Passwort, Öffnung der Krankenakte;
- bei Fehlen eines gültigen Passworts optionales Senden (203) einer E-Mail an den Inhaber des Kontos (203);
- Anzeige (204) des medizinischen Profils für Notfälle, PMU,
- Identifizierung eines vertrauenswürdigen Dritten (205);
- Zugang zu dem vertrauenswürdigen Dritten und Erhalt eines Ersatzschlüssels (206);
- Übertragung des Ersatzschlüssels (207) an den genannten gesicherten Server;
- Prüfung (208) des Ersatzschlüssels und, bei Übereinstimmung mit den in der Krankenakte eingetragenen Schlüsseln, Öffnung der Krankenakte.

4. Verfahren nach Anspruch 3, **gekennzeichnet dadurch, dass** der Zugang zu der digitalen Krankenakte sich aus dem Empfang, durch den gesicherten Server, einer elektronischen Nachricht ergibt, wie eine SMS oder eine E-mail, die von dem eigenen System aus gesendet wird, das dem genannten vertrauenswürdigen Dritten zugeordet ist.

5. Verfahren nach Anspruch 3, **gekennzeichnet dadurch, dass** die Öffnung der digitalen Krankenakte sich aus dem Empfang, durch besagten gesicherten Server, eines Sprachanrufs ergibt, der vom genannten vertrauenswürdigen Dritten erhalten wird.

6. Verfahren nach Anspruch 3, **gekennzeichnet dadurch, dass** der Zugang zu dem genannten gesicherten Server über besagte Speicherkarte oder USB-Karte bei Misserfolg der Passwortprüfungsprozedur zur Sendung einer elektronischen Nachricht an den oder an einen der vertrauenswürdigen Dritten führt, die vorher von besagtem Inhaber der Speicherkarte angegeben wurden.

7. Verfahren nach Anspruch 3, **gekennzeichnet dadurch, dass** es folgende Phasen umfasst:
- Einführung und Ablesung (701) einer nicht personenbezogenen Karte, die ein medizinisches Profil für Notfälle, PMU, umfasst, das keine medizinischen und keine vertraulichen Informationen umfasst, sowie eine Verbindung zum Zugang zu dem genannten Server sowie zu einem oder mehreren vertrauenswürdigen Dritten;
- Anzeige (702) des medizinischen Profils für Notfälle auf einer Vorrichtung zur Verarbeitung der Daten der genannten Krankenakte für dringliche Behandlung;
- Zugang (703) zu dem genannten gesicherten Server (50);
- Prüfung des Passworts (704) und Öffnung der Krankenakte bei Übereinstimmung des Passworts;
- bei falschem Passwort, Prüfung (705) der Authentizität des Systems, das den Zugang anfordert, als einem Notdienst angehörend;
- Identifizierung (706) eines vertrauenswürdigen Dritten;
- Übertragung einer Nachricht an den vertrauenswürdigen Dritten (707), die einen Ersatzschlüssel anfordert;
- Erhalt (708) des besagten Ersatzschlüssels und Rückgewinnung einer Identifizierungsinformation des genannten vertrauenswürdigen Dritten;
- Benutzung (708, 709) besagter Information, um auf den geschützten Bereich des genannten entfernten Servers zuzugreifen.

8. Verfahren zur Erzeugung einer digitalen Krankenakte, die dazu bestimmt ist, auf einem lösbaren Träger gespeichert zu werden, wie einer Speicherkarte, mit einem Speicherraum sowie einem Computerprogramm, das auf besagtem Träger installiert ist, wobei besagtes Verfahren folgende Phasen umfasst:
- Übertragung (1010) einer Anfrage an einen externen Server mit dem Ziel, einen Schlüssel zu erhalten oder einen Code zur Aktivierung des genannten Computerprogramms, besagte Anfrage ermöglicht eine administrative Erfassung beim genannten Server;
- Empfang (1020) des Schlüssels oder des genannten Codes zur Aktivierung, der von besagtem Server erzeugt wurde;
- Start (1030) des genannten Computerprogramms, so dass der Patient eine medizinische Fragebogenakte erfassen kann, die entworfen wurde, um die Sammlung seiner persönlichen medizinischen Daten zu gestatten, und deren Speicherung in einer Datenbank, die in dem genannten Speicherträger angeordnet ist, **gekennzeichnet dadurch, dass**:
- alle erfassten Daten sind je einem Charakter zugeordnet, der ebenfalls vom Patienten erfasst wird, um die Einführung der Daten in ein medizinisches Profil für Notfälle zu gestatten oder nicht, das von besagtem Träger aus frei zugänglich ist, und
- besagtes persönliche medizinische Dringlichkeitsprofil, das dem Patienten entspricht, wird erzeugt;
- zum Beispiel in einem elektronischen Format, das in mehreren Sprachen verfügbar sein kann, wobei dieses persönliche medizinische Dringlichkeitsprofil nur die vom Patienten genehmigten Daten umfasst.

9. Verfahren nach Anspruch 8, **gekennzeichnet dadurch, dass** die vertraulichen Informationen der Krankenakte über einen indirekten Zugang zu der vertraulichen und/oder personenbezogenen digitalen Krankenakte über einen vertrauenswürdigen Dritten gelesen werden können.

10. Verfahren nach Anspruch 8 oder 9, **gekennzeichnet dadurch, dass** besagter Träger eine Verbindung zu besagtem gesicherten Server umfasst, wodurch ein Zugang zu den personenbezogenen und vertraulichen Daten möglich wird, die auf besagtem Server gespeichert sind, über eine Passwortprüfungsprozedur.

11. Verfahren nach Anspruch 8, **gekennzeichnet dadurch, dass** es die folgenden Phasen umfasst:
- Schaffung (101) eines Kontos und Erzeugung eines Benutzernamens und eines Passworts;
- Schaffung (102) eines Verwaltungsfragebogens, der personenbezogene Verwaltungsinformationen umfasst;
- Schaffung (103) eines medizinischen Fragebogens, um medizinische Daten zu sammeln, eventuell mit Anhängen oder Anlagen, die diese Daten validieren;
- Erzeugung (105) einer Synthese der erfassten Daten, über den medizinischen Fragebogen, in Zusammenhang mit einem Klassifizierungswerkzeug, das dazu bestimmt ist, jedem der Daten einen vertraulichen Charakter zuzuweisen oder nicht;
- Angabe (106) eines oder mehrerer vertrauenswürdiger Dritter;
automatische Erzeugung eines medizinischen Profils für Notfälle, PMU, das nur nicht personenbezogene Daten und als nicht vertraulich klassifizierte umfasst, wobei besagtes medizinische Profil für Notfälle in einem nicht geschützten Bereich gespeichert ist, der von einem äußeren Träger aus zugänglich ist, der besagten Benutzernamen umfasst, die anderen personenbezogenen und/oder vertraulichen Daten sind in einem geschützten Bereich des genannten gesicherten Servers gespeichert, der mittels des Benutzernamens und des Passworts zugänglich ist;
besagtes medizinische Profil für Notfälle umfasst außerdem einen Link zum Zugang zu einem vertrauenswürdigen Dritten, zum Erhalt eines Ersatzschlüssels, der bei Fehlen eines Passworts oder jeglichen anderen technischen Mittels zur Ablesung der vertraulichen und/oder personenbezogenen Krankenakte verwendbar ist.

12. Verfahren nach Anspruch 11, **gekennzeichnet dadurch, dass** der Ersatzschlüssel ein Ausdruck des Willens des genannten vertrauenswürdigen Dritten ist, den Zugang zu der Krankenakte zu gestatten, der die Form einer elektronischen Nachricht annimmt, wie SMS oder E-mail, oder eines Sprachanrufs, oder einer elektronischen Nachricht, die durch eine Anwendung von dem System des genannten Dritten aus übertragen wird.

13. Elektronischer Speicherträger für eine digitale Krankenakte, der einen Speicherraum für besagte digitale Krankenakte umfasst, sowie ein Computerprogramm, das auf besagtem Träger installiert ist, angepasst zur Durchführung der Verfahren nach einem der Ansprüche 8 bis 12, wobei besagter Träger folgendes umfasst:
- Mittel zur Übertragung einer Anfrage an einen externen Server mit dem Ziel, einen Schlüssel zu erhalten oder einen Code zur Aktivierung des genannten Computerprogramms, wobei besagte Anfrage eine administrative Erfassung beim genannten Server ermöglicht;
- Mittel, um besagtes Computerprogramm zu starten, wodurch dem Patienten die Erfassung einer medizinischen Fragebogenakte ermöglicht wird, die zur Sammlung seiner persönlichen medizinischen Daten entworfen wurde, und zu deren Speicherung in einer Datenbank, die in dem genannten Speicherträger angeordnet ist, wobei die Erfassung der Zuweisung eines vertraulichen Charakters oder nicht an jede der vom Inhaber der Krankenakte erfassten Informationen zugeordnet ist;
- Mittel, um automatisch ein persönliches medizinisches Dringlichkeitsprofil entsprechend dem Patienten zu erzeugen, zum Beispiel in einem elektronischen Format, das in mehreren Sprachen verfügbar sein kann, und nur die Daten enthält, denen ein nicht vertraulicher Charakter zugeschrieben wurde.

14. Träger nach Anspruch 13, **gekennzeichnet dadurch, dass** er Mittel zum indirekten Zugang zu der vertraulichen und/oder personenbezogenen digitalen Krankenakte über einen vertrauenswürdigen Dritten umfasst.

15. Speicherträger in Form eines Karte, die elektronische Schaltkreise und Speichermittel umfasst, wodurch die Durchführung der in den Ansprüchen 1 bis 12 beschriebenen Prozeduren möglich wird, sowie eine lokale Software in Verbindung mit einem Server zur Umsetzung der Ansprüche in lokalem Modus mit installierter Anwendung.

## Claims

1. A method of generating a digital medical file stored on a secure server (50) and accessible from a first system (10) via a communication network, said digital medical file including nominative and confidential data, said method comprising the automatic generation of an emergency medical file, PMU, devoid of any nominative and confidential information and intended to be stored on a physical support handled by the user, the method comprising the steps of :
- creating (101) an account and generating an identifier and a password;
- creating (102) an administrative questionnaire containing nominative administrative information;
- creating (103) a medical questionnaire for collecting medical data, possibly accompanied by enclosures or attachments validating these data;
- generating (105) a summary of the data entered via the medical questionnaire, in connection with a classification tool intended to let the user authorize or not the introduction of each piece of data inputted into an Emergency Medical Profile which may be freely accessed from said support;
**characterized in that** the method further comprises the steps of:
- designating (106) one or more trusted third parties;
- generating automatically said Emergency Medical Profile, PMU, comprising only non-nominative and data classified as non-confidential by the user, said Emergency Medical Profile being stored into an unprotected area and accessible from said physical support, the other nominative and/or confidential data being stored into a protected area of said secure server accessible by means of the identifier and the password;
wherein said Emergency Medical Profile further comprises a link allowing access to a trusted third party for obtaining a usable substitution key in the absence of a password or any other technical means allowing reading the confidential and/or nominative medical file.

2. The method according to claim 1, **characterized in that** the substitution key results from an expression of the will of said trusted third party to allow access to the medical file, taking the form of an electronic message, such as SMS or email, or a voice call, or an electronic message transmitted by an application from the system of said third party voice.

3. A method for accessing a digital medical file stored on a secure server (50) and accessible from a first system (10) via a communication network, said digital medical file including nominative and confidential data created by the method defined in claim 1, said method comprising the steps of :
- accessing (200) to said secure server (50) by means of a physical support comprising an identifier to the exclusion of any password;
- presenting an identifier and opening (201) a session with said secure server (50);
- checking said password (202) and, in case of valid password, opening the medical file;
- in the event of a invalid password, optionally transmitting (203) an email to the account holder (203);
- displaying (204) the Emergency Medical Profile, PMU,
- identification of a trusted third party (205);
- accessing to the trusted third party and obtaining a substitution key (206);
- transmitting the substitution key (207) to said secure server;
- checking (208) said substitution key and, in case of concordance with the keys registered in the medical file, opening the medical file.

4. The method according to claim 3 **characterized in that** the access to the digital medical file results from the receipt by the secure server of an electronic message, such as a SMS or an email, transmitted from the system owned by said trusted third party.

5. The method according to claim 3, **characterized in that** the opening of the digital medical file results from the reception by said secure server of a voice call received from said trusted third party.

6. The method according to claim 3, **characterized in that** the access to said secure server via said memory card or USB card causes, in case of a failure of said password checking procedure, the transmission of an electronic message to the one or one of the trusted third parties previously designated by said holder of the memory card.

7. The method according to claim 3 **characterized in that** it comprises the steps of:
- inserting and reading (701) a non-nominative card including an Emergency Medical Profile, PMU, containing no medical information and no confidential information, as well as a link allowing access to said server as well as to one or more trusted third parties;
- displaying (702) the Emergency Medical Profile on a data processing device of said emergency medical file;
- accessing (703) said secure server (50);
- checking the password (704) and opening the medical file in case of concordance of the password being input;
- in case of an invalid password, checking (705) the authenticity of the system requiring access, as belonging to an authentic emergency service;
- identifying (706) a trusted third party;
- transmitting a message to the trusted third party (707) requesting a substitution key;
- obtaining (708) said substitution key and retrieving identification information from said trusted third party;
- using (708, 709) of said information to access the protected area to said remote server.

8. A method of generating a digital medical file intended to be stored on a removable medium, such as a memory card, comprising a storage area and a computer program residing on said medium, said method comprising the steps of:
- transmitting (1010) a request to an external server for the purpose of obtaining an activation key or code from said computer program, said request having the purpose of allowing an administrative registration with said server;
- receiving (1020) the key or said activation code generated by said server;
- starting (1030) said computer program allowing the patient to enter a medical questionnaire file designed to allow the collection of his personal medical data, and its storage into a database arranged within said storage support, **characterized in that** each data being input is associated with a flag also input by the patient for authorizing or not the incorporation of such data into a Emergency Medial Profile which is freely accessible from said support, and
- wherein said personal Emergency Medical profile which corresponds to the user is generated, for instance in an electronic format that can be available in several languages, wherein said Emergency Medical Prfole only includes data which have been authorized by the patient.

9. The process according to claim 8 **characterized in that** the access to the confidential information of the medical file can be read via indirect access to the confidential and/or nominative digital medical file via a trusted third party.

10. The method according to the claim 8 or 9, **characterized in that** said support comprises a link to said secure server allowing access to the personal and confidential data stored on said server via a password checking procedure.

11. The method according to claim 8, **characterized in that** it comprises the steps of:
- creating (101) an account and generating an identifier and a password;
- creating (102) an administrative questionnaire containing nominative administrative information;
- creating (103) a medical questionnaire for collecting medical data, possibly accompanied by enclosures or attachments validating these data;
- generating (105) a summary of the data entered via the medical questionnaire, in connection with a classification tool intended to assign to each individual data a confidential nature or not;
- designating (106) one or more trusted third parties;
- generating automatically an Emergency Medical Profile, PMU, comprising only non-nominative data being classified non-confidential data, said Emergency Medical Profile being stored in an unprotected area and accessible from an external medium comprising said identifier, the other nominative and/or confidential data being stored in a protected area of said secure server accessible by means of the identifier and the password;
Wherein said Emergency Medical Profile further comprises a link allowing access to a trusted third party for obtaining a usable substitution key in the absence of a password or any other technical means allowing reading the confidential and/or nominative medical file.

12. The method according to Claim 11, **characterized in that** the substitution key results from an expression of the said trusted third party's will to allow access to the medical file, taking the form of an electronic message, such as SMS or email, or a voice call, or an electronic message transmitted by an application from the system of said third party voice.

13. An electronic storage support for a digital medical file comprising a storage space for said digital medical file, as well as a computer program located on said support, adapted for implementing the methods defined in one of Claims 8 to 12, said support comprising:
- means for transmitting a request to an external server for the purpose of obtaining a key or an activation code of said computer program, said request allowing an administrative registration with said server;
- means for receiving the key or said activation code generated by said server;
- means for starting said computer program enabling the patient to enter a Medical Questionnaire file designed to allow the collection of his personal medical data, and the storage of these data into a database arranged within said storage support, the inputting of the data being associated with the assignment of a confidential flag or not to each piece of information entered by the holder of the medical file;
- means for automatically generating an emergency personal medical profile corresponding to the patient, for example in an electronic format that may be available in several languages, comprising only the data to which a non-confidential flag has been assigned.

14. The support according to claim 13 **characterized in that** it comprises means for indirect access to the confidential and / or nominative digital medical file via a trusted third party.

15. The support taking the form of a card comprising electronic circuits and storage means for implementing the procedures described in claims 1 to 26, and a local software in connection with a server for proposing the claims in a local mode with embedded application.
